# EUROPEAN PATENT APPLICATION

(11) **EP 4 378 399 A1**
(43) Date of publication of application: **05.06.2024**
(21) Application number: 23213509.5
(22) Date of filing: 30.11.2023
(51) Int. Cl.: A61B 17/02, A61B 17/70, A61B 17/04, A61B 17/34, A61B 17/86, A61B 90/50, A61B 17/00

(54) **SURGICAL RETRACTOR SYSTEM AND COMPONENTS**

(30) Priority: 30.11.2022 US 202263428861 P; 24.02.2023 US 202363486847 P; 29.11.2023 US 202318523337
(71) Applicant: Nuvasive, Inc., San Diego, CA 92121 (US)
(72) Inventor: LU, Chunquing, San Diego, CA 92121 (US); KNICHEL, Kelli, San Diego, CA 92121 (US); RIEMHOFER, Byron, San Diego, CA 92121 (US); PATEL, Ashish, San Diego, CA 92121 (US); ALACBAY, Lito, San Diego, CA 92121 (US); BROOKE, Douglas, San Diego, CA 92121 (US); MIKELS, Garrett, San Diego, CA 92121 (US); TAMBEY, Prashant, San Diego, CA 92121 (US); SERRA, Michael, San Diego, CA 92121 (US); ESSAYAN, Gregory, San Diego, CA 92121 (US); FEDON, Shane, San Diego, CA 92121 (US); LILLIG, Steven, San Diego, CA 92121 (US); COLE, Douglas, San Diego, CA 92121 (US); FLORIMO, Sam, San Diego, CA 92121 (US); LAFFOON, Stephen, San Diego, CA 92121 (US); KENNEDY, Neil, San Diego, CA 92121 (US); POOJARY, Brijesh, San Diego, CA 92121 (US); YAM, David, San Diego, CA 92121 (US)
(74) Representative: Morabito, Sara

(57) **Abstract**

Medical devices and methods for using medical devices are disclosed. An example system (100) includes a lateral retractor assembly (112) and a stabilizer releasably coupled to the retractor assembly (112). The retractor assembly (112) is configured to establish an operative corridor to the spine of a prone patient, wherein the stabilizer is configured to maintain the retractor assembly (112) relative to the patient. The stabilizer may include a first stabilizer (1810) configured to engage intervertebral disc tissue and a second stabilizer (1820) configured to engage vertebral tissue. The first and second stabilizers may be or may include stabilizers or stabilizer assemblies configured to releasably engage a retractor blade of the retractor assembly (112).

## Description

### CROSS-REFERENCE TO RELATED APPLICATIONS

This application claims priority to US Provisional App. No. 63/428,861, filed November 30, 2022, and to US Provisional App. No. 63/486,847, filed February 24, 2023, the entire contents of which are each hereby expressly incorporated by reference into this disclosure as if set forth in its entirety herein.

### BACKGROUND

There are a wide variety of surgical medical devices. Some of these devices include surgical retractors, surgical retractor systems, surgical retractor stabilization systems, and the like. Of the known surgical medical devices, each has certain advantages and disadvantages. There is an ongoing need to provide alternative surgical medical devices.

### BRIEF SUMMARY

This disclosure provides design, material, manufacturing method, and use alternatives for medical devices. An example assembly may include a shim body and a guide coupled to the shim body, wherein the shim body may be a surgical shim body configured to releasably couple to a retractor blade of a retractor assembly, and wherein the guide may be configured to receive an attachment component and direct a distal end of the attachment component to vertebral tissue of a subject when the retractor blade is forming a surgical corridor in the subject

Alternatively or additionally to any of the embodiments in this section, the guide may be offset from a central longitudinal axis of the shim body.

Alternatively or additionally to any of the embodiments in this section, the guide may be fixed relative to the shim body.

Alternatively or additionally to any of the embodiments in this section, the guide may be configured to translate inward and outward relative to shim body.

Alternatively or additionally to any of the embodiments in this section, the guide may be configured to pivot relative to the shim body about an axis perpendicular to a longitudinal axis of the shim body.

Alternatively or additionally to any of the embodiments in this section, the guide may have a proximal end and a distal end and a guide tube may extend at least part of a length from the proximal end to the distal end.

Alternatively or additionally to any of the embodiments in this section, the guide has a proximal end, a distal end, and threads proximate the proximal end.

Alternatively or additionally to any of the embodiments in this section, the guide may have a proximal end and a distal end, and the proximal end of the guide may be proximal of a distal end of the shim body and the distal end of the guide may be distal of the distal end of the shim body.

Alternatively or additionally to any of the embodiments in this section, the distal end of the shim body may be configured to engage a proximal end of an intradiscal shim coupled to the retractor blade at a location distal of a location at which the shim body is releasably coupled to the retractor blade.

Alternatively or additionally to any of the embodiments in this section, the assembly may include the attachment component, and the attachment component may be configured to releasably couple to the guide.

Alternatively or additionally to any of the embodiments in this section, the attachment component may be configured to releasably engage the vertebral tissue of the subject to stabilize the retractor assembly relative to movement of a spine of the subject while the retractor assembly is forming a lateral surgical corridor within the subject in a prone position.

Alternatively or additionally to any of the embodiments in this section, the attachment component may be threaded.

Alternatively or additionally to any of the embodiments in this section, the attachment component may include threads at a distal end of the attachment component, the threads at the distal end of the attachment component are configured to releasably engage the vertebral tissue of the subject.

Alternatively or additionally to any of the embodiments in this section, the attachment component may include threads at a location proximal of a distal end of the attachment component, the threads at the location proximal of the distal end of the attachment component are configured to engage threads on the guide.

Alternatively or additionally to any of the embodiments in this section, the attachment component may include a first set of threads at a distal end of the attachment component and a second set of threads at a location proximal of the first set of threads.

Alternatively or additionally to any of the embodiments in this section, the first set of threads may have a first threaded configuration and the second set of threads may have a second threaded configuration.

Alternatively or additionally to any of the embodiments in this section, the first set of threads may be configured to releasably engage threads of the guide, and when the first set of threads are releasably engaged with the threads of the guide, a distal end of the second set of threads may be covered by the guide.

Alternatively or additionally to any of the embodiments in this section, the guide may include a proximal end defining an inner diameter of a guide tube of the guide tube, and a proximal end of the attachment component may define a first outer diameter greater than the inner diameter of the guide tube.

Alternatively or additionally to any of the embodiments in this section, the attachment component may include a second outer diameter that is less than the inner diameter of the guide tube and a proximal end of the guide may be configured to be adjustably spaced from a transition between the first outer diameter and the second outer diameter of the attachment component when the attachment component is engaging the vertebral tissue of the subject.

An example system may include a retractor assembly configured to establish an operative corridor to a spine of a subject, a first stabilizer releasably coupled to the retractor assembly and configured to engage intervertebral disc tissue of the subject while the retractor assembly is establishing the operative corridor, and a second stabilizer releasably coupled to the retractor assembly and configured to engage vertebral tissue of the subject while the retractor assembly is establishing the operative corridor.

Alternatively or additionally to any of the embodiments in this section, the first stabilizer may include a first shim, the second stabilizer may comprise a second shim, and a distal end of the second shim may be configured to engage a proximal end of the first shim.

Alternatively or additionally to any of the embodiments in this section, the second stabilizer may include a shim body and a guide.

Alternatively or additionally to any of the embodiments in this section, the retractor assembly may include a retractor blade, and the shim body may be configured to engage the retractor blade.

Alternatively or additionally to any of the embodiments in this section, the guide may be coupled to the shim body and is offset from a longitudinal axis of the retractor blade.

Alternatively or additionally to any of the embodiments in this section, the guide may be configured to be fixed relative to the shim body.

Alternatively or additionally to any of the embodiments in this section, the guide is configured to be adjusted relative to the shim body and the retractor blade.

Alternatively or additionally to any of the embodiments in this section, the second stabilizer may include an attachment component, and the attachment component may be configured to releasably couple to the guide.

Alternatively or additionally to any of the embodiments in this section, the attachment component may be configured to releasably engage the vertebral tissue of the subject to stabilize the retractor assembly relative to movement of the spine of the subject while the retractor assembly is establishing a lateral surgical corridor within the subject in a prone position.

Alternatively or additionally to any of the embodiments in this section, the guide, the shim body, and the retractor assembly may be configured to adjust relative to an axis of the attachment component when the attachment component is releasably engage with the vertebral tissue of the subject.

An example method may include engaging a first stabilizer with an intervertebral disc tissue of a subject in a prone position, the first stabilizer is coupled with a retractor assembly, forming a lateral surgical corridor within the subject using the retractor assembly, and engaging a second stabilizer with vertebral tissue of the subject, the second stabilizer is coupled with the retractor assembly.

Alternatively or additionally to any of the embodiments in this section, the second stabilizer may include a shim body configured to engage a blade of the retractor assembly, a guide coupled to the shim body, and an attachment component configured to be inserted through the guide and engaged with the vertebral tissue of the subject.

Alternatively or additionally to any of the embodiments in this section, the method may include engaging a first set of threads of the attachment component with threads of the guide, and a distal end of a second set of threads of the attachment component may be covered by the guide when the first set of threads of the attachment component are engaged with the threads of the guide.

Alternatively or additionally to any of the embodiments in this section, the method may include engaging the shim body with the blade of the retractor assembly while the second set of threads of the attachment component are covered by the guide.

Alternatively or additionally to any of the embodiments in this section, engaging the shim body with the blade of the retractor assembly may occur after engaging the first stabilizer with the intervertebral disc tissue.

Alternatively or additionally to any of the embodiments in this section, the method may include threading the attachment component through the threads of the guide and into the vertebral tissue of the subject.

Alternatively or additionally to any of the embodiments in this section, the attachment component may be threaded into the vertebral tissue of the subject until a predetermined distance is between a distal shoulder of a head of the attachment component and a proximal end of a guide tube of the guide.

Alternatively or additionally to any of the embodiments in this section, the method may include rotating the attachment component to disengage the attachment component from the vertebral tissue of the subject.

Alternatively or additionally to any of the embodiments in this section, the method may include withdrawing the second stabilizer from the lateral surgical corridor.

Alternatively or additionally to any of the embodiments in this section, the withdrawing the second stabilizer from the lateral surgical corridor may include disengaging the shim body from the blade of the retractor assembly.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
FIG. 1 is a perspective view depicting the creation of a lateral access corridor formed with a surgical access system via a lateral approach through the side of a patient to a target disc space;
FIG. 2 is a partial cross-sectional view of an example tissue retractor assembly forming a lateral access corridor with a surgical access system via a lateral approach through the side of a patient to a target disc space;
FIG. 3 illustrates an example surgical shim in a closed configuration;
FIG. 4 illustrates the surgical shim shown in FIG. 3 in an open configuration;
FIG. 5 illustrates the example surgical shim shown in FIGS. 3-4 in an open configuration positioned adjacent to two vertebral bodies;
FIG. 6 illustrates a portion of an example surgical retractor stabilization system;
FIG. 7 illustrates a cross-sectional view of the example surgical retractor stabilization system taken along line 7-7 of FIG. 6;
FIG. 8 illustrates an example surgical retractor stabilization system;
FIG. 9 illustrates an example surgical retractor stabilization system;
FIG. 10A is a front view of another example surgical shim which may be utilized with a surgical retractor stabilization system;
FIG. 10B is a side view of another example surgical shim which may be utilized with a surgical retractor stabilization system;
FIG. 11A is a front view of another example surgical retractor stabilization system;
FIG. 11B is a back view of the example surgical retractor stabilization system of FIG. 11A;
FIG. 11C is a side view of the example surgical retractor stabilization system of FIG. 11A;
FIG. 11D is a side view of the example surgical retractor stabilization system of FIG. 11A;
FIG. 12 is a cross-sectional view of an example surgical retractor stabilization system;
FIG. 13 illustrates an example surgical retractor stabilization system;
FIG. 14 is a cross-sectional view of an example surgical retractor stabilization system;
FIG. 15A is a side view of an example surgical retractor stabilization system;
FIG. 15B is a back view of an example surgical retractor stabilization system;
FIG. 16 illustrates another example surgical retractor stabilization system;
FIG. 17 illustrates an example surgical retractor stabilization system;
FIG. 18 illustrates an example surgical retractor stabilization system;
FIG. 19 illustrates an example anchor member;
FIG. 20 illustrates an example anchor member;
FIGS. 21-22 illustrates an example anchor member in a first position and a second position;
FIG. 23 illustrates an example surgical retractor stabilization system;
FIG. 24 illustrates an example surgical retractor stabilization system;
FIG. 25 illustrates an example surgical retractor stabilization system;
FIG. 26 illustrates an example surgical retractor stabilization system;
FIG. 27 illustrates an example surgical retractor stabilization system;
FIG. 28 illustrates an example surgical retractor stabilization system;
FIG. 29 illustrates an example surgical retractor stabilization system;
FIG. 30 illustrates a top view of an example surgical retractor stabilization system;
FIG. 31 illustrates an exploded view of an example surgical shim assembly;
FIG. 32 illustrates a perspective view of the example surgical shim assembly of FIG. 31;
FIG. 33 illustrates a front side view of the example surgical shim assembly of FIG. 31;
FIG. 34 illustrates a back side view of the example surgical shim assembly of FIG. 31;
FIG. 35 illustrates a right side view of the example surgical shim assembly of FIG. 31;
FIG. 36 illustrates a left side view of the example surgical shim assembly of FIG. 31;
FIG. 37 illustrates a top view of the example surgical shim assembly of FIG. 31;
FIG. 38 illustrates a bottom view of the example surgical shim assembly of FIG. 31;
FIG. 39 illustrates an example shim having a guide configured to translate;
FIG. 40 illustrates an example shim having a guide configured to pivot;
FIGS. 41A-41C illustrate an example method of inserting an attachment component of a surgical shim assembly; and
FIG. 42A-42C illustrate an example method of removing an attachment component of a surgical shim assembly.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the spirit and scope of the disclosure.

### DETAILED DESCRIPTION

The spine provides mobility, support, and balance. For example, the spine protects the nerves of the spinal cord, which convey commands from the brain to the rest of the body, and convey sensory information from the nerves below the neck to the brain. Even minor spinal injuries can be debilitating to the patient, and major spinal injuries can be catastrophic. The loss of the ability to bear weight or permit flexibility can immobilize the patient. Even in less severe cases, small irregularities in the spine can put pressure on the nerves connected to the spinal cord, causing devastating pain and loss of coordination.

A number of medical procedures, such as spinal surgeries, may be performed to address a variety of spinal conditions. For example, spinal fusion is a medical procedure performed to reduce the symptoms of damaged spinal discs, or for any pathology that would suggest direct spinal decompression as a treatment. The primary goals of spinal fusion procedures are to provide stability between the vertebrae on either side of the damaged disc and to promote natural fusion of those adjacent vertebrae.

A spinal fusion procedure may be performed via minimally invasive or minimal access techniques (in contrast to traditional "open" surgical techniques). In some instances, open surgical techniques may be generally undesirable in that they typically require large incisions and high amounts of tissue displacement to gain access to a surgical target site, which may produce concomitantly high amounts of pain, lengthened hospitalization (increasing health care costs), and high morbidity in the patient population. Less-invasive surgical techniques (including so-called "minimal access" and "minimally invasive" techniques), however, may be preferred because they involve accessing the surgical target site via incisions of substantially smaller size with greatly reduced tissue displacement requirements. This, in turn, reduces the pain, morbidity and cost associated with such procedures.

Some less-invasive surgical techniques may include utilizing surgical systems and methods optimized for creating a lateral access operative corridor to the lumbar spine. Improvement of surgical systems and methods employed while utilizing a lateral access approach has the potential to further reduce operative time, expand applications for the lateral approach and increase surgeon adoption of the procedure, all of which may ultimately benefit patients by providing more opportunity for minimally invasive surgical correction of their ailments.

In some examples, spinal surgery via a lateral access approach may utilize a surgical retractor system to manipulate one or more surgical retractor blades to create an operative corridor through a lateral side of a patient lying in a prone position. However, utilizing a surgical retractor system in conjunction with a lateral access approach may result in drooping and a lack of stabilization of the surgical retractor system due to gravitational forces acting on the surgical retractor system as the system extends laterally out from a subject. Further, a clinician may be required to pass one or more instruments through an operative corridor established by the surgical retractor system, whereby the clinician may inadvertently hit the surgical retractor system as the clinician passes the one or more instruments within the operative corridor. The instruments hitting the surgical retractor system may apply forces on the retractor system in a variety of directions, thereby resulting in a lack of stabilization of the surgical retractor system. As such, use of surgical retractor systems in conjunction with a lateral access approach may require the use of surgical retractor stabilization systems designed to support and counter the gravitational forces and impaction forces placed on the surgical retractor system during the procedure.

A need exists for improvements relating to surgical retractor stabilization systems and methods. The systems and methods described herein are directed at addressing these needs. The present application describes surgical retractor systems (including surgical retractor stabilization instruments to be utilized in conjunction with surgical retractor assemblies) and related instruments and methods for performing minimally invasive spinal surgery.

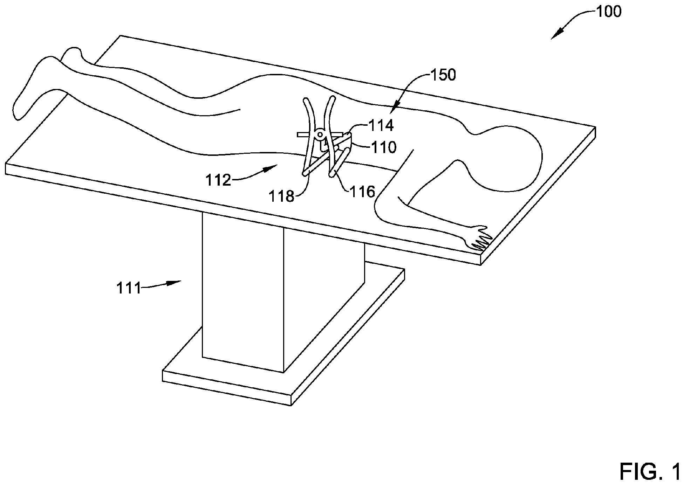

FIG. 1 is a perspective view of a portion of an example surgical system 100. A patient 150 lying in a prone position on an operating table 111 (e.g., located in an operating suite) is illustrated in FIG. 1. Further, a surgical retractor assembly 112 that has been positioned along a lateral side of the patient 150 to establish an operative corridor to a surgical target site within the patient 150 is illustrated in FIG. 1. As discussed above, because the surgical retractor assembly 112 extends laterally out from the patient 150, gravitational forces may act on the surgical retractor assembly 112 in a manner that causes the surgical retractor assembly 112 to inadvertently adjust relative to the patient 150 and as a result, adjust a positioning of the operative corridor created by the surgical retractor assembly 112.

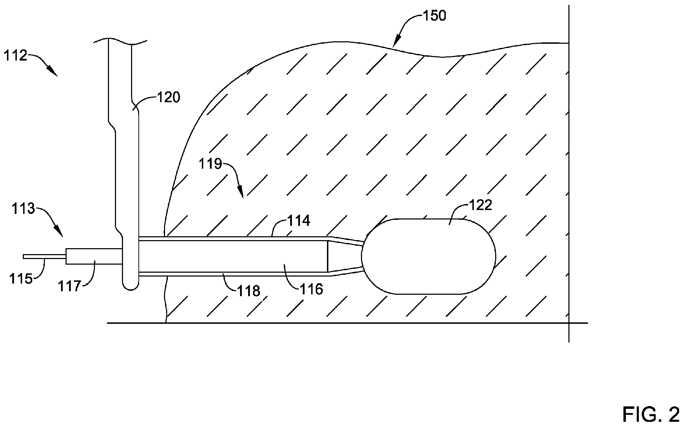

With reference to FIG. 2, a discussion of an example lateral access methodology is provided. With the patient 150 positioned on their stomach (e.g., prone position), a portion of the surgical retractor assembly 112 may be advanced through an incision into the retroperitoneal space, and then through the psoas muscle until the targeted spinal site (e.g. the disc space between a pair of adjacent vertebral bodies) is reached.

In some examples, a dilation system 113 may be utilized to establish a pathway for the surgical retractor system to be inserted into the patient 150. The dilation system 113 may include a sequential dilation system 113 with an initial dilator 115 and one or more additional dilators 117 of increasing diameter. In some instances, the initial dilator 115 is preferably advanced to the target site first, and then each of the additional dilators 117 of increasing diameter may be advanced in turn over the previous dilator. A k-wire (not shown) may be advanced to the target site and docked in place (for example, by inserting the k-wire into the vertebral disc) prior to, in concurrence with, or after advancing the initial dilator 115 to the target site. With the sequential dilation system 113 positioned adjacent the target site (and optionally docked in place via the k-wire), the retractor assembly 112 may then be advanced to the target site over the sequential dilation system 113.

According to the example shown, the surgical retractor assembly 112 may include retractor blades 114, 116, 118 and a body 120. In some examples, the retractor assembly 112 may be advanced over the dilation system 113 such that the center retractor blade 114 is the posterior most blade. The sequential dilation system 113 may then be removed and the retractor assembly 112 may be opened to expand the operative corridor. For example, the retractor blades 114, 116 and 118 may be separated relative to one another (as illustrated in FIG. 1) to establish the lateral access operative corridor through which instruments and implants may be advanced to the target site. Although described and shown herein with reference to a generally lateral approach to a spinal surgical target site (with the first blade 114 being the "posterior" blade, the second blade 116 being the "cranial-most" blade, and the third blade 118 being the "caudal-most" blade), it will be appreciated that the retractor assembly 112 of the present invention may find use in any number of different surgical approaches, including generally posterior, generally postero-lateral, generally anterior and generally antero-lateral. Additionally, it will be appreciated that any number of procedures may be performed on the spine through the lateral access corridor (e.g. the surgeon may perform a fusion procedure, a total disc replacement, a corpectomy, etc.).

As discussed herein, in some examples the surgical retractor assembly 112 may be used in conjunction with one or more surgical retractor stabilization systems to provide support to the surgical retractor assembly 112 when utilized during a lateral access procedure or other suitable procedures. For example, via one or more surgical retractor stabilization systems, one or more of the retractor blades 114, 116, 118 may be fixed in a position relative to the spine prior to opening the retractor blades 114, 116, 118 to establish the operative corridor.

In some cases, fixing one or more retractor blades 114, 116, 118 in a position relative to the spine may be accomplished by attaching a shim (e.g., a stabilizer)to one of the retractor blades 114, 116, 118 and inserting the distal end of the shim into the disc space. For example, following (or before) the opening of the retractor blades 114, 116, 118, a shim (which, in one example, may be slidably engaged with the posterior retractor blade 114) may be advanced such that a distal end region of the shim is positioned within the posterior region of the disc space. Positioning the distal end region of the shim within the posterior region of the disc space before retraction of the blades 114, 116, 118 may help ensure that the posterior retractor blade 114 will not move posteriorly, or otherwise, during the retraction process, even though the other retractor blades 116, 118 are able to move and thereby create an operative corridor of a desired or adjustable size.

Fixing the posterior retractor blade 114 may rigidly couple the posterior retractor blade 114 in a fixed relation relative to the vertebral bodies adjacent the disc space. Additionally, rigidly coupling the posterior retractor blade 114 in a fixed relation relative to the vertebral bodies may generally stabilize the surgical retractor assembly 112 with respect to gravitation forces acting to pull the assembly 112 in an anterior (e.g., downward) direction relative to the patient. In other words, rigidly coupling the posterior retractor blade 114 in a fixed relation relative to the vertebral bodies of the spine may prevent the retractor assembly 112 (including the retractor blades 114, 116, 118) from shifting (e.g., drooping, dropping, sagging, dipping, etc.) downward or undesirably adjusting in response to tool impaction as a clinician is attempting to insert one or more instruments through an operative corridor established by the blades 114, 116, 118.

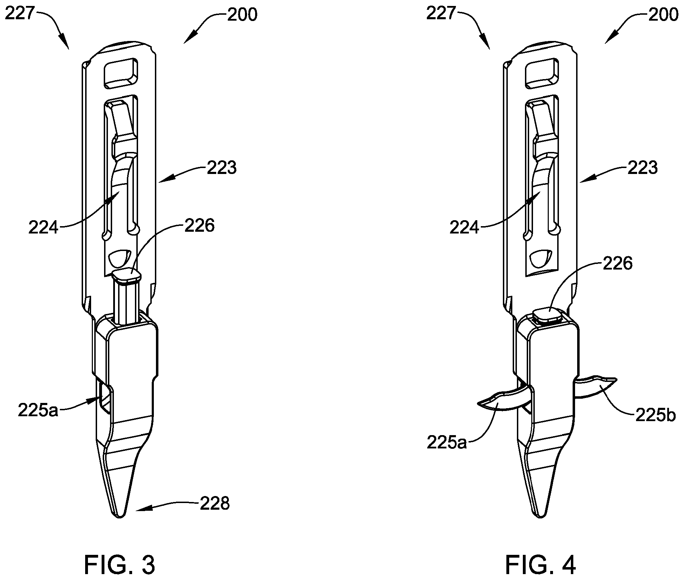

A shim 200 (e.g., a stabilizer), for example as depicted in FIG. 3, may be used with the surgical retractor assembly 112 to facilitate stabilizing a position of the surgical retractor assembly 112 during a lateral access procedure. The example shim 200 may include a generally rigid body portion 223, a proximal end region 227 and a distal end region 228. The distal end region 228 may include a generally tapered shape designed to be inserted into the disc space of the spine. Additionally, the shim 200 may include a tab element 224 capable of engaging with one or more corresponding grooves (or other similar feature) along an inner-facing surface of the retractor blades 114, 116, 118. In some examples, the tab element 224 may also be equipped with an enlarged projection which engages within corresponding grooves provided along an inner surface of the retractor blades 114, 116, 118.

The shim 200 illustrated in FIG. 3 may include a first tack member 225a and a second tack member 225b (hidden from view in FIG. 3, but shown in FIG. 4), each of which may be coupled to a piston 226. The first tack member 225a and the second tack member 225b may be couple to the piston 226 with any suitable coupling mechanism. In one example, a pin or other suitable coupling mechanism may extend through the first tack member 225a and the second tack member 225b, and couple to the piston 226 within the body portion 223 of the shim 200.

Further, actuation of the piston 226, as illustrated when comparing FIG. 3 to FIG. 4, may force each of the first tack member 225a and the second tack member 225b to splay laterally outward. In other words, depressing the piston 226 may cause the first tack member 225a and the second tack member 225b to pivot about the coupling mechanism (e.g., a pin or other suitable coupling mechanism) and shift from a position within the shim 200 to a position in which the distal ends (e.g., the distal tip) of each of the first tack member 225a and the second tack member 225b extend away from the lateral edge of the body portion 223 for engagement with a vertebral disc or adjacent vertebrae to secure the shim 200 and an attached surgical retractor assembly 112 relative to the spine of the patient 150.

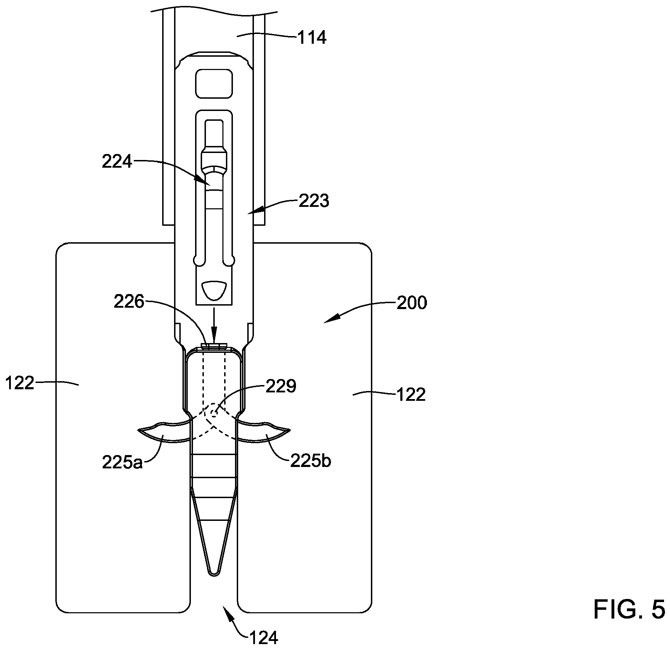

In some cases, the shim 200 may be coupled to the posterior retractor blade 114, as illustrated in FIG. 5. While the shim 200 is coupled to the posterior retractor blade 114 in FIG. 5 and may facilitate using the retractor assembly 112 in a procedure using a lateral approach, it is contemplated that the shim 200 may be coupled to any additional retractor blade of the retractor assembly 112). In use, the distal end region 228 of the shim 200 may be positioned within the disc space 124 located between two adjacent vertebral bodies 122. Once the distal end region 228 is positioned within the disc space 124, the piston 226 may be actuated toward the distal end region 228 of the shim 200, as illustrated in FIG. 5, thereby forcing each of the first tack member 225a and the second tack member 225b laterally outward such that distal ends of the first tack member 225a and the second tack member 225b engage a respective one of the vertebral bodies 122. Engagement of the first tack member 225a and the second tack member 225b with the vertebral bodies may stabilize the retractor assembly 112 relative to the vertebral bodies 122. Additionally, FIG. 5 illustrates that the first take member 225a and the second tack member 225b may be coupled via a pin connection 229.

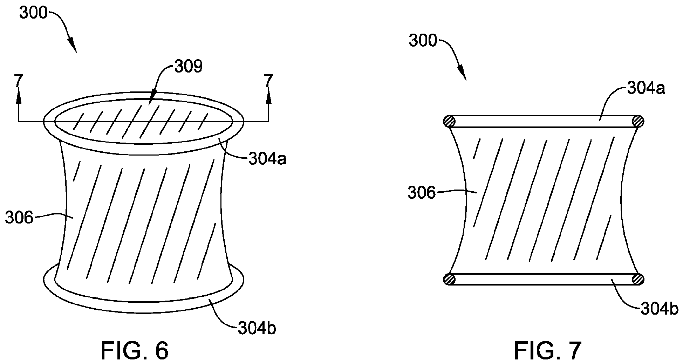

An expandable retractor 300 of an example surgical retractor stabilization system is illustrated in FIG. 6. The retractor 300 may include a first support ring 304a, a second support ring 304b (e.g., positioned at an opposite end from an end at which the first support ring 304a is positioned), and an expandable cylindrical body portion 306 extending between the first support ring 304a and the second support ring 304b. The expandable retractor 300 may radially expand from a collapsed configuration to an expanded configuration, thereby defining an opening 309 (e.g., tunnel, passage, operative corridor) which may be utilized to pass one or more surgical instruments therethrough.

The expandable body portion 306 of the retractor 300 may be formed from a flexible polymer membrane, a wire mesh structure, a combination polymer-wire structure or from any other suitable material capable of being able to shift between a semi-rigid cylindrical structure and a collapsed configuration. Each of the first support ring 304a and the second support ring 304b may be or may include at least a semirigid polymer ring. Each of the body portion 306, the first support ring 304a and the second support ring 304b may be capable of shifting between a collapsed configuration and an expanded configuration (such as that illustrated in FIG. 6). Additionally, in the expanded configuration, the retractor 300 may include enough radial strength to retract a patient's skin, thereby maintaining an operative corridor to a surgical target site.

FIG. 7 illustrates a cross-sectional view of the retractor 300 taken along line 7-7 of FIG. 6. FIG. 7 illustrates the retractor 300 in an expanded configuration. As described herein, FIG. 7 illustrates the expandable body portion 306 extending between the first support ring 304a and the second support ring 304b.

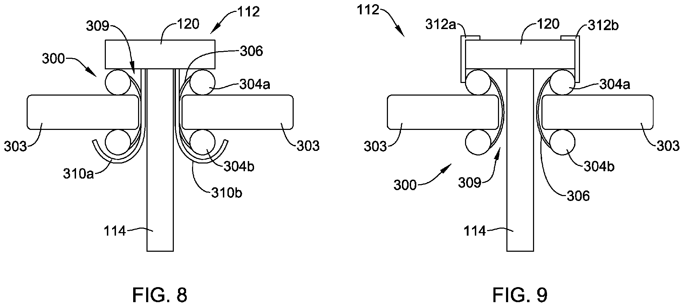

The expandable retractor 300 is depicted in cross-section in FIG. 8 and positioned across an abdominal wall 303 of a patient. The expandable retractor 300 may retract the abdominal wall 303 of the patient, thereby permitting a retractor blade (e.g., the posterior retractor blade 114 or other suitable retractor blade) of a surgical retractor assembly 112 to extend through the opening 309 of the retractor 300.

In some examples, the surgical retractor assembly 112, the expandable retractor 330 may be utilized with or as part of a retractor stabilization system, as depicted in FIGS. 8 and 9. In one example, the surgical retractor assembly 112 depicted in FIG. 8 may include one or more engagement members (e.g., first engagement 310a and second engagement member 310b) which may be coupled to one or more other features of the surgical retractor assembly 112. For example, the first and second engagement members 310a, 310b depicted in FIG. 8 may be coupled (e.g., attached, engaged, etc.) to the retractor body 120 or one or more the retractor blades 114, 116, 118. FIG. 8 illustrates each of the first and second engagement members 310a/310b attached to the retractor body 120.

Additionally, each of the first and second engagement members 310a, 310b may pass through the opening 309 of the expandable retractor 300, as depicted in FIG. 8. After passing through the opening 309 of the expandable retractor 300, in one example, each of the first and second engagement members 310a, 310b may fold over the second support ring 304b to engage the second support ring 304b. When folded over to engage the second support ring 304b, the first and second engagement members 310a, 310b (via their attachment to the body 120 of the retractor assembly 112), may stabilize the retractor assembly 112 relative to a target surgical site.

Another example of a retractor stabilization system utilized in conjunction with the retractor assembly 112 is depicted in FIG. 9. The retractor stabilization system illustrated in FIG. 9 may be similar in form and function to the surgical stabilization system described with respect to FIG. 8. For example, FIG. 9 illustrates the expandable retractor 300 positioned across the abdominal wall 303 of a patient. The expandable retractor 300 may retract the abdominal wall 303 of the patient, thereby permitting a retractor blade 114 (e.g., a proximal retractor blade 114) of a surgical retractor assembly 112 to extend through the opening 309 of the retractor 300.

Further, the retractor stabilization system shown in FIG. 9 may include, among other components, first and second engagement members 312a, 312b which are coupled to the body 120 of the retractor assembly 112 and releasably coupled to the first support ring 308a of the retractor 300. Unlike the first and second engagement members 310a, 310b of the retractor stabilization system of FIG. 8, the first and second engagement members 312a, 312b may not be configured to extend through the opening 309 of the retractor 300. Rather, the first and second engagement members 312a, 312b may be positioned outside of the patient. In some examples, the first and second engagement members 312a, 312b may include an "L" shape whereby one side of the first and second engagement member 312a, 312b attaches to the body 120 of the retractor assembly 112 and the other side of the first and second engagement member 312a, 312b attaches (e.g., a clip or other suitable coupling mechanism) to the first attachment support ring 308a of the retractor 300. When attached to both the body 120 of the retractor assembly 112 and the first attachment support ring 308a, the first and second engagement members 312a, 312b may stabilize the retractor assembly 112 relative to a target surgical site.

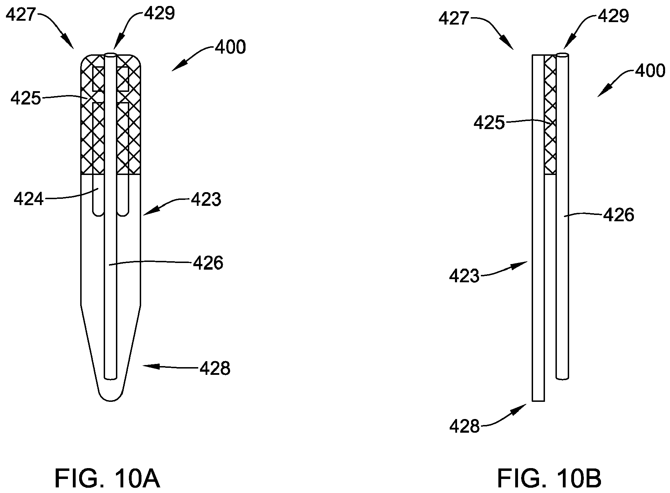

A front view of an example shim 400 (e.g., a stabilizer) is illustrated in FIG. 10A, which may be utilized in conjunction with a retractor assembly (e.g., the retractor assembly 112 described herein or other suitable retractor assembly). The shim 400 may include a generally rigid body portion 423, a proximal end region 427 and a distal end region 428. The distal end region 428 may include a generally tapered shape designed to be inserted into the disc space between adjacent vertebrae of the spine. In some cases, the shim 400 may include a tab element 424 (similar to the tab element 224 described with respect to FIG. 3), capable of engaging with one or more corresponding grooves (or other similar feature) along an inner-facing surface of one or more of the retractor blades 114, 116, 118 of the retractor assembly 112. In some examples, the tab member 424 may also be equipped with an enlarged projection which engages within corresponding grooves provided along an inner surface of the retractor blades 114, 116, 118.

The shim 400 depicted in FIG. 10A may further include a tubular member 426 coupled to the shim 400 via a connection member 425. The connection member 425 may be positioned between the front surface of the shim 400 and the tubular member 426, as depicted in FIG. 10B. Additionally, the tubular member 426 depicted in FIGS. 10A and 10B may extend from the proximal end region 427 to the distal end region 428 of the shim 400. In some cases, the tubular member 426 may include a lumen 429 through which a K-wire may extend. As discussed herein, a k-wire (not shown) may be advanced to the target site and docked in place (for example, by inserting the k-wire into the vertebral disc) during a pre-dilation step (e.g., a pre-dilation step in which the insertion site is dilated in advance of the full retraction of the retractor blades 114, 116, 118 to establish an operative corridor). In some instances, the k-wire may be inserted through the vertebral disc endplates and into a vertebral body. Similar to that described with respect to the shim depicted in FIG. 3, engagement of the shim 400 with the vertebral bodies adjacent the target tissue site may stabilize the retractor assembly 112 relative to the vertebral bodies.

In some examples, such as that illustrated in FIGS. 10A-10B, the shim 400, the connection member 425 and the tubular member 426 may be constructed as a single, monolithic structure. However, in other examples, the connection member 425 may be formed as a separate structure which may be subsequently attached to the shim 400. In some instances, the connection member 425 and the tubular member 426 may be formed as a monolithic component which is subsequently attached (e.g., snap fit, etc.) to the shim 400.

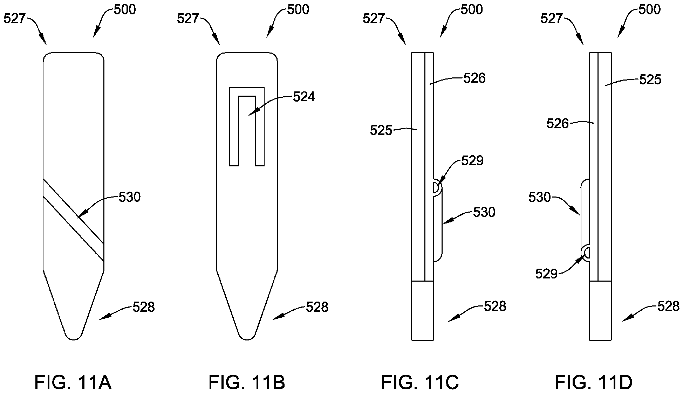

A front view of another example shim 500 (e.g., a stabilizer) is depicted in FIG. 11A, which may be utilized in conjunction with a retractor assembly (such as the retractor assembly 112 described herein or other suitable retractor assembly). The shim 500 may include a generally rigid body portion 523, a proximal end region 527 and a distal end region 528. The distal end region 528 may include a generally tapered shape designed to be inserted into the disc space between adjacent vertebral bodies of the spine.

A back view of the shim 500 is depicted in FIG. 11B. The shim 500, as depicted in FIG. 11B, may include a tab element 524 (similar to the tab element 224 described with respect to FIG. 3), capable of engaging with one or more corresponding grooves (or other similar feature) along an inner-facing surface of the retractor blades 114, 116, 118 of the retractor assembly 112. In some examples, the tab element 524 may also be equipped with an enlarged projection which engages within corresponding grooves provided along an inner surface of the retractor blades 114, 116, 118.

Side views of the shim 500 are depicted in FIGS. 11C and 11D. The shim 500 may be constructed as a two-part shim, as depicted in FIGS. 11C and 11D, whereby the shim 500 includes a first part 525 and a second part 526, the second part 526 being disposed on the first part 525.

In some examples, the first part 525 and the second part 526 may be formed from the same material. In other examples, the first part 525 and the second part 526 may be formed from different materials.

The shim 500 may include a lumen 529 (e.g., aperture, opening, tunnel, passage, channel, etc.) extending diagonally across the shim 500 from one side to the opposite side, as depicted in FIGS. 11C and 11D. A first side opening of the lumen 529 is depicted in FIG 11C and a second side opening of the lumen is depicted in FIG. 11D, wherein the lumen 529 may extend continuously from the first side to the second (opposite) side, but this is not required. In some examples, the lumen 529 may be formed through a raised portion 530 of the second part 526 (e.g., as depicted in FIGS. 11C and 11D). In other words, the shim 500 may include a raised portion 530 that extends diagonally across the shim 500, and whereby the lumen 529 extends through the raised portion 530 diagonally across the shim 500.

Further, a k-wire (not shown) may be advanced to the target site through the lumen 529 and docked in place (e.g., by inserting the k-wire into the vertebral disc or one or more adjacent vertebral bodies) during a pre-dilation step (e.g., a pre-dilation step in which the insertion site is dilated in advance of the full retraction of the retractor blades 114, 116, 118 to establish an operative corridor). Similar to that described with respect to the shim of FIG. 3, engagement of the shim 500 with the disc between adjacent vertebral bodies and the k-wire with one or both of the adjacent vertebral bodies proximate the target tissue site may stabilize the retractor assembly 112 relative to the vertebral bodies.

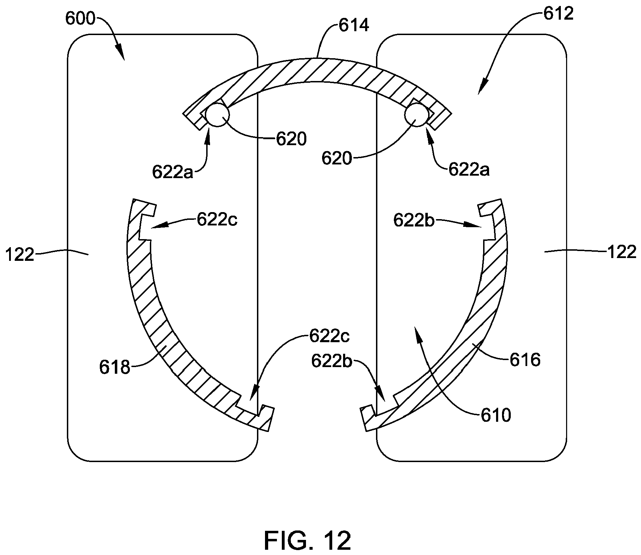

An example retractor stabilization system 600 is depicted in FIG. 12. Example retractor blades 614, 616, 618 are depicted in cross-section in FIG. 12, whereby the retractor blades 614, 616, 618 may be similar in form and function to the retractor blades 114, 116, 118 shown in FIG. 1. In other words, FIG. 12 illustrates a cross-sectional view of the retractor blades 614, 616, 618 in an open configuration to define an operative corridor 610 to a target surgical site.

Further, FIG. 12 illustrates that each of the retractor blades 614, 616, 618 may include one or more channels extending along an inner surface of the retractor blades 614, 616, 618. For example, the posterior retractor blade 614 may include two channels 622a extending along an inner surface of the retractor blade 614, the retractor blade 616 may include two channels 622b extending along an inner surface of the retractor blade 616 and the retractor blade 618 may include two channels 622c extending along an inner surface of the retractor blade 618, but this is not required and other suitable configurations are contemplated. In some cases, the channels 622a, 622b, 622c may extend from a proximal end region of the retractor blade 614, 616, 618, respectively, to a distal end region of the retractor blade 614, 616, 618, respectively.

The channels 622a, 622b, 622c depicted in FIG. 12 may permit a stabilizing pin to be inserted along the channel 622a, 622b, 622c, whereby the stabilizing pin may secure one or more of the retractor blades 614, 616, 618 to a vertebral body 122. For example, a stabilizing pin 620 may be disposed within each of the channels 622a of the posterior retractor blade 614, as depicted in FIG. 12. The channel 622a may be designed such that the stabilizing pins 620 may slide longitudinally within the channel 622a, yet the channel 622a may be designed to prevent the stabilizing pin 620 sliding out of the channel 622a, away from the inner surface of retractor blade 614. In some cases, the channels 622b of the retractor blade 616 and the channels 622c of the retractor blade 618 may be similarly designed to allow stabilizing pins inserted therein to slide longitudinally, yet prevent the stabilizing pins from sliding out of the channel, away from the inner surface of the retractor blades.

In some cases, when inserted into a vertebral body 122, the stabilizing pins 620 may stabilize the proximal retractor blade 614 and the retractor assembly 612 relative to the target surgical site. Because the channels may be designed to permit the stabilizing pins 620 to slide longitudinally within the channel 622a while also preventing the stabilizing pins 620 from sliding out of the channel 622a away from the inner surface of the proximal retractor blade 614, the stabilizing pins 620 may be screwed into the vertebral bodies 122 and stabilize the posterior retractor blade 614 and the retractor assembly 612 relative to a target surgical site during a procedure utilizing a lateral approach or during other suitable procedures. Although the posterior retractor blade 614 have two stabilizing pins 620 inserted therein in FIG. 12, it is contemplated that any of the retractor blades 116, 118 may include one or more stabilizing pins disposed within channels 622b, 622c (and thereby be secured to the vertebral bodies 122).

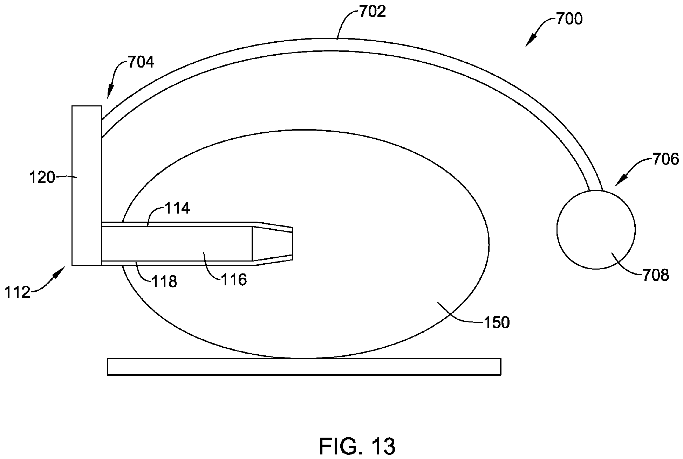

Another example retractor stabilization system is depicted in FIG. 13, which may be utilized in conjunction with the retractor assembly 112. An example counterweight system 700 is depicted in FIG. 13. The counterweight system 700 may include an arm 702 having a proximal end attached to the body 120 of the retractor assembly 112 and a distal end 706 attached to a counterweight 708. As discussed herein, the retractor blades 114, 116, 118 may be inserted into a patient 150 (schematically depicted) to establish an operative corridor, as depicted in FIG. 13.

FIG. 13 further illustrates that the arm 702 may extend from the body 120 of the retractor assembly 112, overtop and across the body of the patient 150 (e.g., the arm may arc overtop of the patient 150). It can be appreciated that the arm 702 may act as a lever arm to offset the downward gravitational force place on the retractor assembly 112. The specific weight of the counterweight 708 may be designed to counterbalance a downward force generated at least in part by a mass of the retractor assembly 112. In some examples, the counterweight 708 and a flex in the arm 702 may be designed to offset the downward gravitational force placed on the retractor assembly 112 such that the retractor assembly 112 (including the retractor blades 114, 116, 118) is stabilized in a generally fixed position. In other examples, the counterweight 708 may hang freely, unattached to another support structure. In yet other examples, the counterweight may be designed to attach to a fixed component of the operating suite (e.g., operating table, wall, rail, pole, etc.).

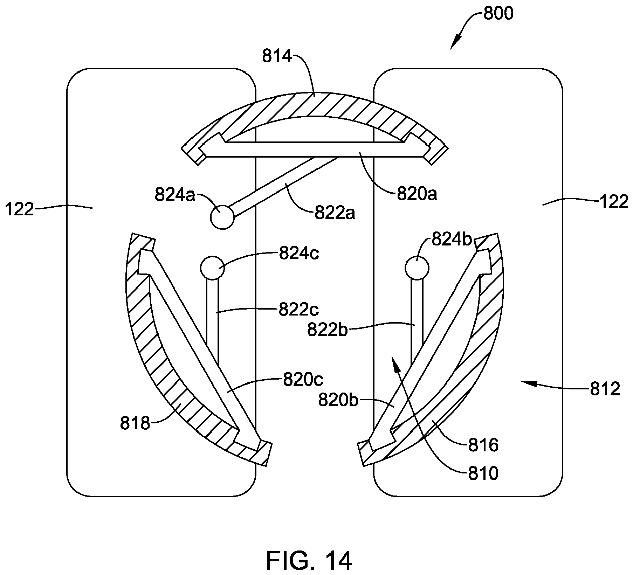

An example retractor stabilization system 800 is depicted in FIG. 14. A cross-sectional view of example retractor blades 814, 816, 818 is depicted in FIG. 14, whereby the retractor blades 814, 816, 818 may be similar in form and function to the retractor blades 114, 116, 118 shown in FIG. 1. The retractor blades 814, 816, 818 depicted in FIG. 14 are in an open configuration to define an operative corridor 810 to a target surgical site.

Each of the retractor blades 814, 816, 818 may include a base member 820a, 820b, 820c attached to an inner surface of each of the retractor blades 814, 816, 818, respectively, as depicted in FIG. 14. Additionally, each of the retractor blades 814, 816, 818 may further include an arm 822a, 822b, 822c, each arm 822a, 822b, 822c having a proximal end extending from a respective base member 820a, 820b, 820c, and a distal end receiving or coupled to a stabilizing screw 824a, 824b, 824c. Each of the stabilizing screws 824a, 824b, 824c may be designed to screw into a vertebral body 122 of a patient, thereby fixedly securing one or more of the retractor blades 814, 816, 818 to a vertebral body 122.

When inserted into a vertebral body 122, one or more of the stabilizing screws 824a, 824b, 824c may stabilize the retractor blade 814, 816, 818 to which the stabilizing screw 824a, 824b, 824c is coupled. The stabilizing screws 824a, 824b, 824c coupled with respective retractor blades 814, 816, 818 may be designed to offset the downward gravitational force placed on the retractor assembly 812 such that the retractor assembly 812 (including the retractor blades 814, 816, 818) may be stabilized in a generally fixed position.

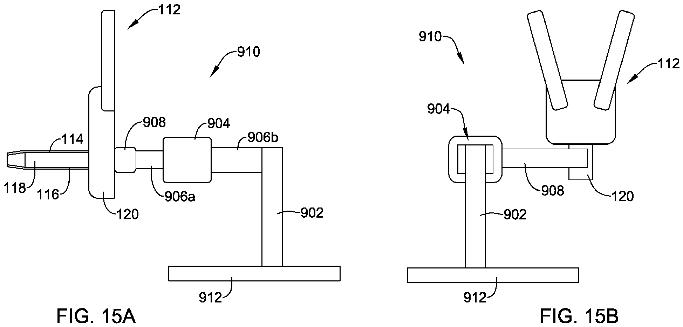

An example retractor stabilization system 900 is depicted in FIGS. 15A and 15B, which may be utilized in conjunction with the retractor assembly 112. The retractor assembly 112 (including the retractor blades 114, 116, 118 and body 120) may be coupled to a depth rack assembly 910. The depth rack assembly 910 may be designed to manipulate the position of the retractor assembly 112 relative to a patient. For example, the depth rack assembly 910 may be designed to permit the retractor assembly 112 to be advanced into or withdrawn from an insertion site of a patient. Further, as discussed herein, the retractor blades 114, 116, 118 may be utilized to establish an operative corridor to a target surgical site within a patient. The depth rack assembly 910 may function to aid in the placement of the retractor blades 114, 116, 118 when establishing the operative corridor.

The depth rack assembly 910 may include a first arm 902 (e.g., an articulating arm or other suitable arm), as depicted in FIG. 15A. The first arm 902 may include a first end designed to attach to a fixed component 912 of the operating suite (e.g., operating table, wall, etc.). The depth rack assembly 910 may also include one or more telescoping members (e.g., a first telescoping member 906a and a second telescoping member 906b, such as telescoping arms, beams, etc.). The first and second telescoping members 906a, 906b may be designed to extend and retract relative to one another. For example, the first and second telescoping members 906a, 906b may be designed to lengthen and shorten a combined length in response to relative movement of one another. Additionally, to the depth rack assembly 910 may include a connector 904. Further, each of first and second telescoping members 906a, 906b may be housed at least partially within or extend through the connector 904. One of the first and second telescoping members 906a, 906b may nest within the other first and second telescoping member 906a, 906b while housed within the connector 904.

Manipulation of the first and second telescoping members 906a, 906b may be utilized to advance or retract the retractor assembly 112 relative to an insertion site of a patient In some examples, the first and second telescoping members 906a, 906b and the connector 904 together may entirely or at least partially define a system, mechanism, assembly, etc. that is designed to advance, retract, or maintain the retractor assembly 112 relative to an insertion site of a patient. For example, the second telescoping member 906b may include a spring designed to exert a force on the first telescoping member 906a, which may push the retractor assembly 112 toward a patient. Further, in some examples, the connector 904 may include a ratchet assembly or other suitable adjustment assembly. The ratchet assembly or other suitable adjustment assembly may be designed to retract the first telescoping member 906a toward the second telescoping member 906b, whereby a spring positioned within the second telescoping member 906b may be compressed. Further, the ratchet assembly or other suitable adjustment assembly positioned within the connector 904 may permit the first telescoping member 906a to be released incrementally, whereby releasing the first telescoping member 906a allows expansion of the spring and advancement of the first telescoping member 906a toward the patient in a controlled manner. When the first telescoping member 906a is at a desired location relative to the second telescoping member 906b, the ratchet assembly or other suitable adjustment assembly may releasably maintain the first telescoping member 906a at the desired location.

In some examples, the rack assembly 910 may further include a second arm 906 designed to laterally offset the retractor assembly 112 relative to the rack member 904 and first arm 902, as depicted in FIG. 15B. The second arm 906 may include a first end coupled to one of the telescoping members 906a, 906b and may also include a second end coupled to the body 120 of the retractor assembly 112.

The depth rack assembly 910 may be designed to offset the downward gravitational force placed on the retractor assembly 112 such that the retractor assembly 112 (including the retractor blades 114, 116, 118) may be stabilized in a generally fixed position. Although not depicted, the depth rack assembly 910 may include one or more locking components to secure the depth rack assembly 910 in a configuration relative to the retractor assembly 112.

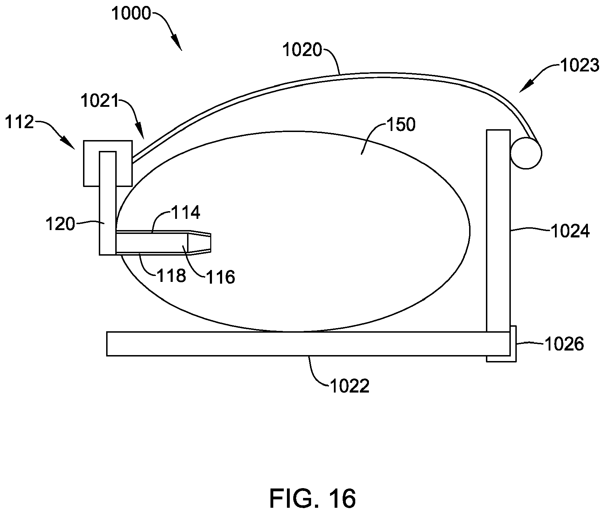

An example retractor stabilization system 1000 is depicted in FIG. 16, which may be utilized in conjunction with the retractor assembly 112. FIG. 16 further illustrates the retractor blades 114, 116, 118 inserted into a patient 150 to establish an operative corridor.

The stabilization system 1000 may include a strap 1020 having a first end 1021 attached to the body 120 of the retractor assembly 112 and a second end 1023 attached to a support member 1024. In some examples, the support member 1024 may include a bolster attached thereto. Additionally, in some examples the support member 1024 may include a releasable attachment member 1026 (e.g., clamp, hook, bracket, clasp, etc.) designed to couple the support member 1024 to a fixed component 1022 of the operating suite. For example, the support member 1024 may include a clamp (e.g., the attachment member 1026), which may be coupled to a table (e.g., the fixed component 1022) on which the patient 150 is positioned, as depicted in FIG. 16.

FIG. 16 further illustrates that the strap 1020 may extend from the body 120 of the retractor assembly 112, overtop and across the body of the patient 150. It can be appreciated that the strap (e.g., a flex of the strap) coupled to the support member 1024 may counterbalance the downward gravitational force place on the retractor assembly 112. In some cases, the strap 1020 may be designed to offset the downward gravitational force placed on the retractor assembly 112 such that the retractor assembly 112 (including the retractor blades 114, 116, 118) is stabilized in a generally fixed position.

In some examples, the second end 1023 of the strap 1020 may not couple to the attachment member 1026, but rather, the second end 1023 of the strap 1020 may be configured to wrap around a torso of the patient 150 and couple to the body 120 of the retractor assembly 112 or, alternatively, couple to the first end 1021 of the strap 1020. The strap 1020 may be tensioned to provide an appropriate force to counterbalance the downward gravitational force place on the retractor assembly 112.

In yet other examples, the second end 1023 of the strap 1020 may not couple to the attachment member 1026, but rather, the second end 1023 of the strap 1020 may be coupled to a counterweight configured to counterbalance a downward force generated at least in part by a mass of the retractor assembly 112. The strap 1020 may be designed to offset the downward gravitational force placed on the retractor assembly 112 such that the retractor assembly 112 (including the retractor blades 114, 116, 118) is stabilized in a generally fixed position.

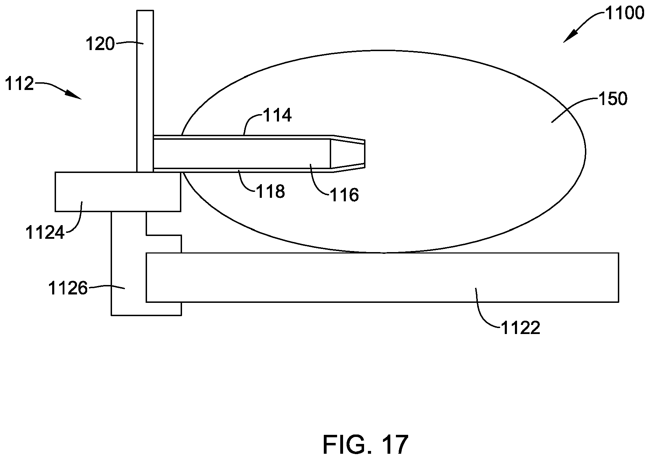

An example retractor stabilization system 1100 is depicted in FIG. 17, which may be utilized in conjunction with the retractor assembly 112. The retractor blades 114, 116, 118 may be inserted into a patient 150 to establish an operative corridor, as depicted in FIG. 17 and discussed herein.

The stabilization system 1100 may include a platform 1124 (e.g., table, stand, scaffold, etc.) designed to support the retractor assembly 112 thereupon. For example, FIG. 17 shows the retractor assembly 112 resting on the platform 1124 while the retractor blades 114, 116, 118 are inserted into the patient 150 to establish an operative corridor. In some examples, the platform 1124 may be coupled to a releasable attachment member 1126 (e.g., clamp, hook, bracket, clasp, etc.) designed to couple the platform 1124 to a fixed component 1122 of the operating suite. For example, FIG. 17 illustrates that the platform 1124 coupled to a clamp (e.g., the attachment member 1126) which is coupled to a table (e.g., a fixed component 1122) on which the patient 150 is positioned. The platform 1124 may be designed to offset the downward gravitational force placed on the retractor assembly 112 such that the retractor assembly 112 (including the retractor blades 114, 116, 118) is stabilized in a generally fixed position.

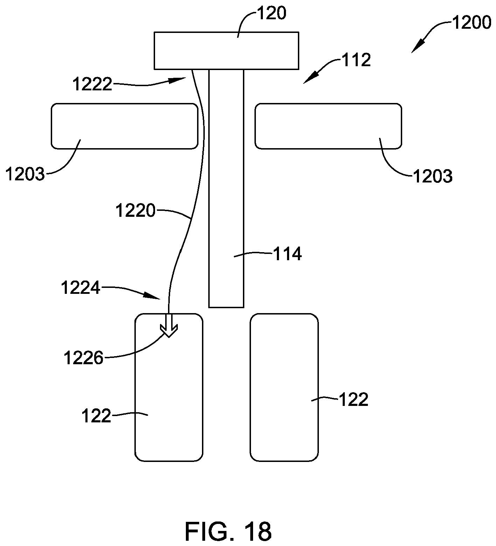

An example retractor stabilization system 1200 is depicted in FIG. 18, which may be utilized in conjunction with the retractor assembly 112. The retractor blade 114 may be inserted into a patient 150 to establish an operative corridor, as depicted in FIG. 18. In some cases, the retractor stabilization system 1200 may include a tether member 1220 having a first end coupled (e.g., attached, engaged, etc.) to the retractor body 120 and a second end 1224 designed to engage a vertebral body 122 of the patient.

In some cases, the tether member 1220 may extend across the skin (e.g., abdominal wall 1203) of the patient, whereby the second end 1224 may engage (e.g., anchor into) a vertebral body 122 of the patient. As will be described in greater detail herein, the second end 1224 of the tether member 1220 may include an anchor member 1226 designed to engage (e.g., anchor into) a vertebral body 122.

In some examples, the tether member 1220 may be formed from a material which permits the tether member 1220 to be placed in tension when the tether member 1220 is coupled to both the retractor body 120 and the vertebral body 122. When coupled to both the retractor body 120 and the vertebral body 122, the tether member 1220 may be designed to offset the downward gravitational force placed on the retractor assembly 112 such that the retractor assembly 112 (including the retractor blades 114, 116, 118) is stabilized in a generally fixed position.

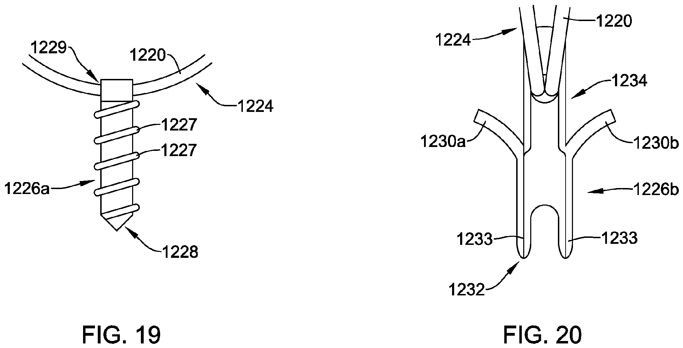

An example anchor member 1226a which may be utilized with the tether member 1220, is depicted in FIG. 19. The anchor member 1226a may be coupled to the second end of the tether member 1220. Further, the anchor member 1226a may include a distal end 1228 which has a generally pointed shape, as depicted in FIG. 19. Additionally, the anchor member 1226a may include one or more threads 1227 designed to permit the anchor to "screw" into a vertebral body 122. Additionally, the anchor member 1226a may include an eyelet 1229 designed to permit the second end 1224 of the tether member 1220 to extend therethrough.

Another example anchor member 1226b which may be utilized with the tether member 1220 is depicted in FIG. 20. The anchor member 1226b may be coupled to the second end 1224 of the tether member 1220. FIG. 20 illustrates that the anchor member 1226b may include a distal end 1232 which includes one or more prongs 1233 designed to pierce a vertebral body 122. In some examples, the prongs 1233 may form a pilot hole in the vertebral body 122.

The anchor member 1226b may include one or more expansion arms 1230a, 1230b designed to pierce and anchor into a vertebral body 122. The one or more expansion arms 1230a, 1230b may be designed to shift from a first configuration (e.g., a delivery configuration) in which the one or more expansion arms 1230a, 1230b are generally parallel to the longitudinal axis of the anchoring member 1226b (for insertion into the patient) and a second configuration in which the one or more expansion arms 1230a, 1230b are extended to engage a vertebral body 122.

Alternatively, the one or more expansion arms 1230a, 1230b may be designed to provide an interference fit of the anchoring member 1226 within the disc space between two adjacent vertebral bodies 122. The one or more expansion arms 1230a, 1230b may be designed to shift from a first configuration (e.g., a delivery configuration) in which the one or more expansion arms 1230a, 1230b are generally parallel to the longitudinal axis of the anchoring member 1226b (for insertion into the patient) and a second configuration in which the one or more expansion arms 1230a, 1230b are extended to provide an interference fit within the disc space between the vertebral bodies 122. Additionally, the anchor member 1226b may include an eyelet 1234 designed to permit the second end 1224 of the tether member 1220 to extend therethrough.

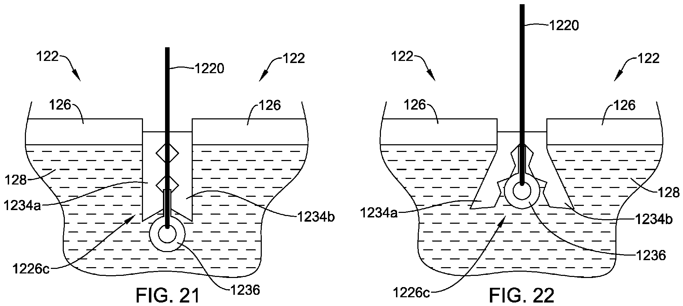

Another example anchor member 1226c which may be utilized with the tether member 1220 is depicted in FIGS. 21 and 22. The anchor member 1226c depicted in FIG. 21 may include a distal eyelet 1236 coupled to the second end 1224 of the tether member 1220. Additionally, the anchor member 1226c may further include a first expandable arm 1234a and a second expandable arm 1234b, as depicted in FIG. 21. In some cases, the tether member 1220 may pass between the first expandable arm 1234a and the second expandable arm 1234b.

The anchor member 1226c depicted in FIGS. 21 is in a first configuration whereby the anchor member 1226c is positioned within the cancellous bone 128 of a vertebral body 122 after being inserted through a hole formed in the compact bone 126 of the vertebral body 122. In the first configuration, the first expandable arm 1234a and the second expandable arm 1234b are in a retracted position (e.g., have not been expanded).

The anchor member 1126c depicted in FIG. 22 is in a second configuration whereby the anchor member 1226c is positioned within the cancellous bone 128 of a vertebral body 122 after being inserted through a hole formed in the compact bone 126 of the vertebral body 122. The eyelet 1236 depicted in FIG. 22 has been retracted between the first expandable arm 1234a and the second expandable arm 1234b, whereby the retraction of the eyelet 1236 causes the first expandable arm 1234a and the second expandable arm 1234b to expand outward (e.g., the first expandable arm 1234a and the second expandable arm 1234b are driven laterally outward away from the tether member 1220). The expansion of the first expandable arm 1234a and the second expandable arm 1234b, as depicted in FIG. 22, may prevent the anchor member 1226c from being retracted back through the vertebral bodies 122.

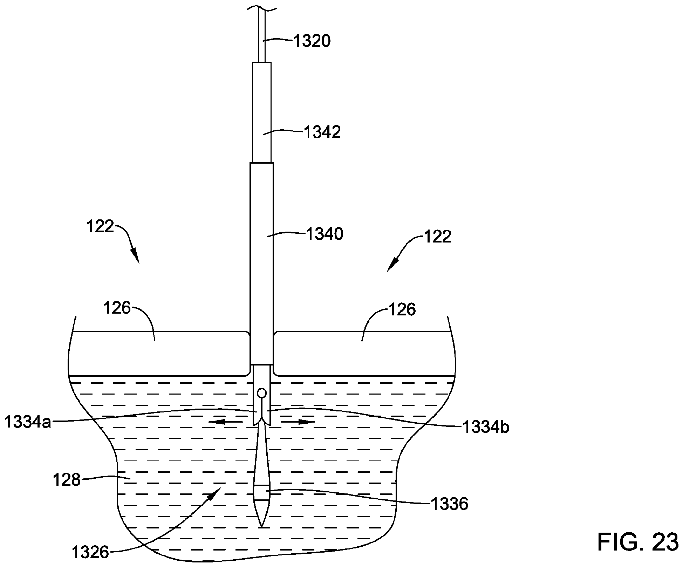

An example anchor system 1326 is depicted in FIG. 23. The anchor system 1326 is positioned within the cancellous bone 128 of a vertebral body 122 after being inserted through a hole formed in the compact bone 126 of the vertebral body 122. The anchor system 1326 may include a distal eyelet 1336 coupled to a distal end of the tether member 1320. In some examples, the tether member 1320 shown in FIG. 23 may include a wire. The anchor system 1326 depicted in FIG. 23 may further include a cannula 1342 which includes a first expandable arm 1334a and a second expandable arm 1334b disposed along a distal end of the cannula 1342. In some cases, the cannula 1342 may include a lumen which permits the tether member 1320 to extend therein. Accordingly, the tether member 1320 may translate within the lumen of the cannula 1342.

The cannula 1342 may extend within a lumen of an outer cannula 1340 and the cannula 1342 may translate within the lumen of the outer cannula 1340. In some examples the outer cannula 1340 may be coupled to a shim, but this is not required. Additionally, similar to the anchor member 1226c described herein, the first expandable arm 1334a and a second expandable arm 1334b disposed along a distal end of the cannula 1342 may be positioned adjacent to vertebral bodies 122.

The tether member 1320 may pass between the first expandable arm 1334a and the second expandable arm 1334b, as depicted in FIG. 23. Similar to the anchor member 1226c described herein, the eyelet 1336 may be retracted between the first expandable arm 1334a and the second expandable arm 1334b, whereby the retraction of the eyelet 1336 expands the first expandable arm 1334a and the second expandable arm 1334b outward (e.g., the first expandable arm 1334a and the second expandable arm 1334b are driven laterally outward away from the tether 1320). It can be appreciated that the expansion of the first expandable arm 1334a and the second expandable arm 1334b may prevent the anchor system 1326 from being retracted back through the vertebral bodies 122.

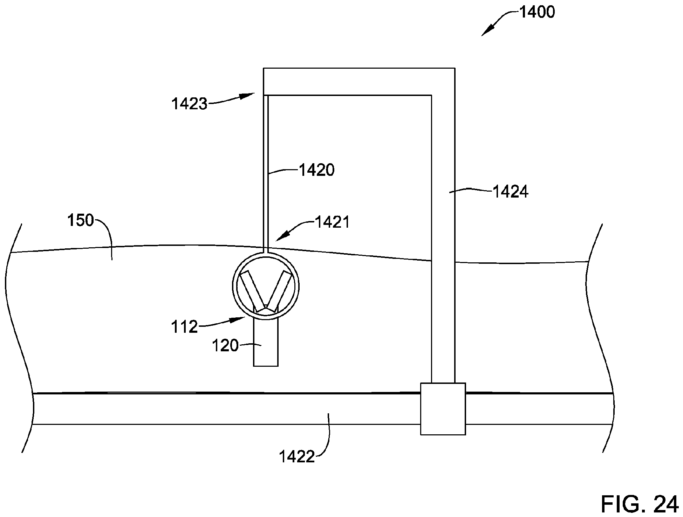

An example retractor stabilization system 1400 which may be utilized in conjunction with the retractor assembly 112 is depicted in FIG. 24. As discussed herein, the retractor assembly 112 being utilized to establish an operative corridor into a patient 150.

The stabilization system 1400 may include a support member 1420 (e.g., rope, wire, chain, etc.) having a first end 1421 coupled to the body 120 of the retractor assembly 112 and a second end 1423 coupled to a support arm 1424. Further, in some examples, the support arm 1424 may include a releasable attachment member 1426 (e.g., clamp, hook, bracket, clasp, etc.) designed to couple the support arm 1424 to a fixed component 1422 of the operating suite. In one example, the support arm 1424 may include a clamp (e.g., the attachment member 1426) which is coupled to a table (e.g., the fixed component 1422) on which the patient 150 is positioned, as depicted in FIG. 24. In some examples the support arm 1424 may be directly attached to a fixed component 1422 of the operating suite. For example, the support arm 1424 may be welded (or attached using any suitable attachment technique) directly to the fixed component 1422, but this is not required.

The stabilization system 1400 may permit the retractor assembly 112 to hang from the support member 1420 or be pulled up from the support member 1420. Accordingly, the stabilization system 1400 may be designed to offset the downward gravitational force placed on the retractor assembly 112 such that the retractor assembly 112 (including the retractor blades 114, 116, 118) is stabilized in a generally fixed position.

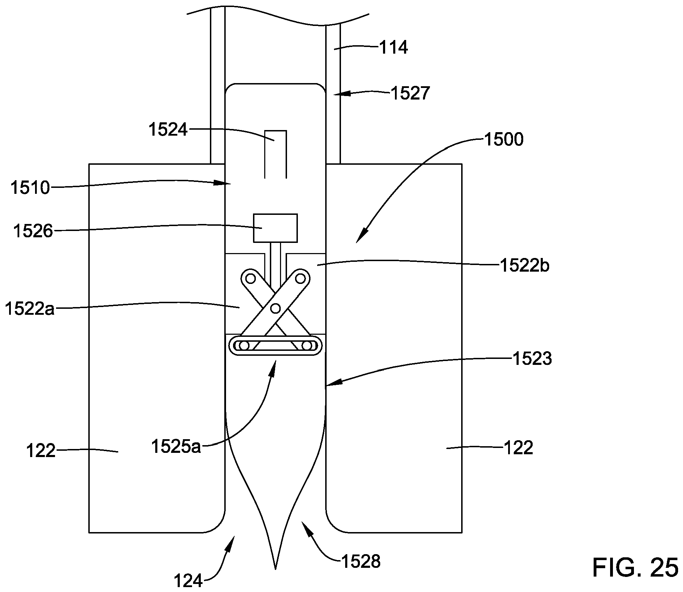

An example retractor stabilization system 1500 which may be utilized in conjunction with the retractor assembly 112 is depicted in FIG. 25. The stabilization system 1500 may include a shim 1510 (e.g., a stabilizer), among other suitable components. The shim 1510 may include a generally rigid body portion 1523, a proximal end region 1527, and a distal end region 1528. The distal end region 1528 may include a generally tapered shape designed to be inserted into the disc space of the spine. Additionally, the shim 1510 may include a tab element 1524 capable of engaging with one or more corresponding grooves (or other similar feature) along an inner-facing surface of the retractor blades 114, 116, 118. In some examples, the tab element 1524 may also be equipped with an enlarged projection which engages within corresponding grooves provided along an inner surface of the retractor blades 114, 116, 118.

The stabilization system 1500 may include a first engagement member 1522a and a second engagement member 1522b coupled to the shim 1510 via a linkage assembly 1525a. Additionally, the stabilization system 1500 may further include a screw 1526 coupled to the linkage assembly 1525a. Actuation of the screw 1526 may actuate the linkage assembly 1525a, whereby actuation of the linkage assembly 1525a may force each of the first engagement member 1522a and the second engagement member 1522b to spread laterally outward.

The distal end region 1528 of the shim 1510 may be positioned within the disc space 124 located between two adjacent vertebral bodies 122, as depicted in FIG. 25. Actuation of the screw 1526 may force each of the first engagement member 1522a (e.g., a first tack member) and the second engagement member 1522b (e.g., a second tack member) laterally outward such that each of the first engagement member 1522a and the second engagement member 1522b engage one of the vertebral bodies 122. Engagement of the first engagement member 1522a and the second engagement member 1522b with the vertebral bodies 122 may stabilize the retractor assembly 112 relative to the vertebral bodies 122.

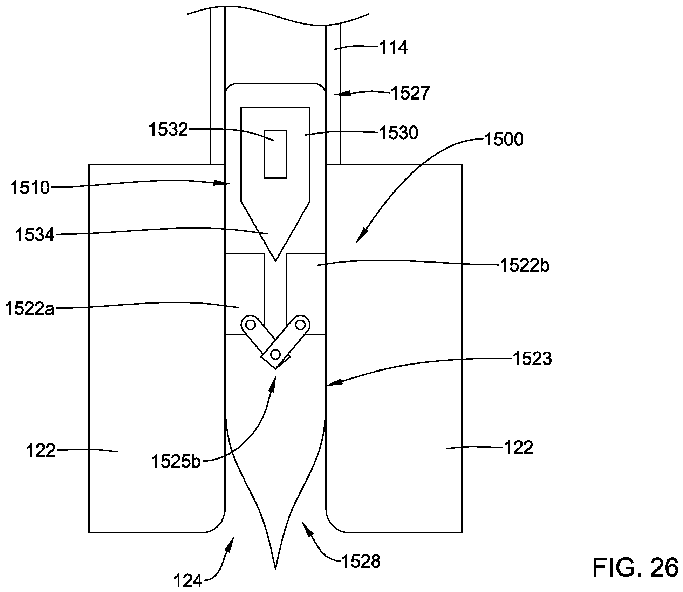

A variation of the retractor stabilization system 1500 which may be utilized in conjunction with the retractor assembly 112 is depicted in FIG. 26. The stabilization system 1500 shown in FIG. 26 may include a shim 1510, among other suitable components. The shim 1510 may include a generally rigid body portion 1523, a proximal end region 1527, and a distal end region 1528. The distal end region 1528 may include a generally tapered shape designed to be inserted into the disc space of the spine. Additionally, the shim 1510 may include a tab element 1524 (shown in FIG. 26) capable of engaging with one or more corresponding grooves (or other similar feature) along an inner-facing surface of the retractor blades 114, 116, 118. In some examples, the tab element 1524 may also be equipped with an enlarged projection which engages within corresponding grooves provided along an inner surface of the retractor blades 114, 116, 118.

The stabilization system 1500 shown FIG. 26 may include a first engagement member 1522a and a second engagement member 1522b coupled to the shim 1510 via a linkage assembly 1525b. Additionally, the stabilization system 1500 shown in FIG. 26 may further include a wedge 1530 coupled to the shim 1510. The wedge 1530 may be aligned with the linkage assembly 1525b. The wedge 1530 may include an actuation member 1532 coupled thereto. Actuation of the actuation member 1532 may be designed to drive the wedge 1530 toward and between the first engagement member 1522a and the second engagement member 1522b, whereby driving the wedge 1530 between the first engagement member 1522a and the second engagement member 1522b may spread the first engagement member 1522a and the second engagement member 1522b laterally outward.

The distal end region 1528 of the shim 1510 may be positioned within the disc space 124 located between two adjacent vertebral bodies 122, as depicted in FIG. 26. Driving the wedge 1530 between the first engagement member 1522a and the second engagement member 1522b may force each of the first engagement member 1522a (e.g., a first tack member) and the second engagement member 1522b (e.g., a second tack member) laterally outward such that each of the first engagement member 1522a and the second engagement member 1522b engage one of the vertebral bodies 122. Engagement of the first engagement member 1522a and the second engagement member 1522b with the vertebral bodies 122 may stabilize the retractor assembly 112 relative to the vertebral bodies 122.

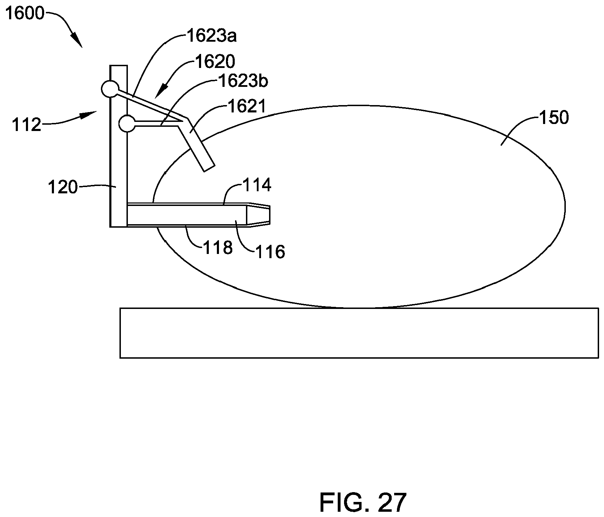

An example retractor stabilization system 1600 which may be utilized in conjunction with the retractor assembly 112 is depicted in FIG. 27. As discussed herein, the retractor assembly 112 may be utilized to establish an operative corridor into a patient 150.

The retractor stabilization system 1600 may include an anchoring assembly 1620. In some instances, the anchoring assembly 1620 may include a first support arm 1623a and a second support arm 1623b. Each of the first support arm 1623a and a second support arm 1623b may include a first end attached to the body 120 of the retractor assembly 112. Additionally, each of the first support arm 1623a and the second support arm 1623b may include a second end which is attached to an anchor pin 1621.

In some examples, the anchor pin 1621 may extend at an angle relative to each of the first support arm 1623a and the second support arm 1623b. Additionally, the anchor pin 1621 may be designed to be anchored into a bone of the patient 150. In one example, the anchor pin 1621 may be designed to anchor into a hip bone of the patient 150, but the anchor pin 1621 may be configured to engage other bone material of the patient 150. Engagement of the anchor pin 1621 with a bone of a patient 150 may stabilize the retractor assembly 112 relative to the operative corridor. Additionally, the anchor pin 1621 may be designed to engage to posterior fixation screws already positioned in the spine.

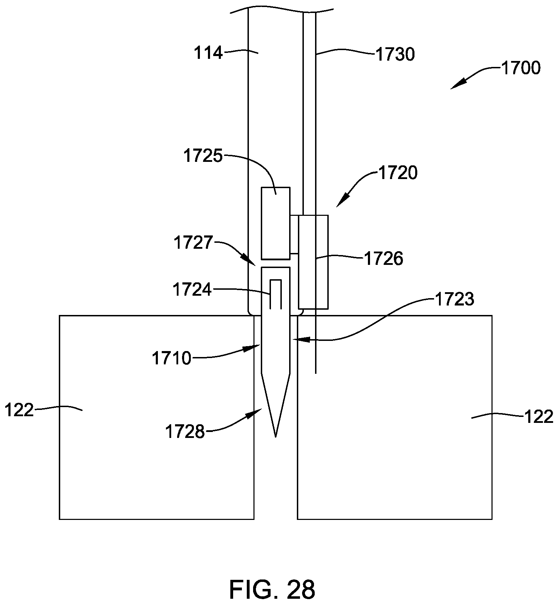

An example retractor stabilization system 1700 which may be utilized in conjunction with the retractor assembly 112 is depicted in FIG. 28. The stabilization system 1700 may include a first shim 1710 (e.g., a first stabilizer). The first shim 1710 may include a generally rigid body portion 1723 (e.g., a shim body), a proximal end region 1727 and a distal end region 1728. The distal end region 1728 may include a generally tapered shape designed to be inserted into the disc space 124 of the spine. Additionally, the first shim 1710 may include a tab element 1724 capable of engaging with one or more corresponding grooves (or other similar feature) along an inner-facing surface of a retractor assembly blade (e.g., one of the retractor blades 114, 116, 118). In some examples, the tab element 1724 may also be equipped with an enlarged projection which engages within a corresponding groove provided along an inner surface of a retractor blade 114, 116, 118 of the retractor assembly 112.

The stabilization system 1700 may include a second shim 1720 (e.g., a second stabilizer). The second shim 1720 may include an attachment region 1725 designed to engage with one or more corresponding grooves positioned along an inner-facing surface of a retractor blade 114, 116, 118 of the retractor assembly 112. Additionally, the second shim 1720 may include an offset portion 1726 which is coupled to the attachment region 1725. The offset portion 1726 of the second shim 1720 may include a lumen extending thru the offset portion 1726 of the second shim 1720. In some examples, an attachment component 1730 (e.g., a k-wire or other suitable attachment component), which may or may not be part of the second shim 1720, may extend through may extend through the lumen of the offset portion 1726 of the second shim 1720. The attachment component 1730 may be advanced to the target site and docked in place (for example, by inserting the k-wire into the vertebral disc) during a pre-dilation step (e.g., a pre-dilation step in which the insertion site is dilated in advance of the insertion of the retractor blades 114, 116, 118 to establish an operative corridor). Engagement of the second shim 1720 with the vertebral bodies 122 adjacent the target tissue site (e.g., via the attachment component 1730) may stabilize the retractor assembly 112 relative to the vertebral bodies.

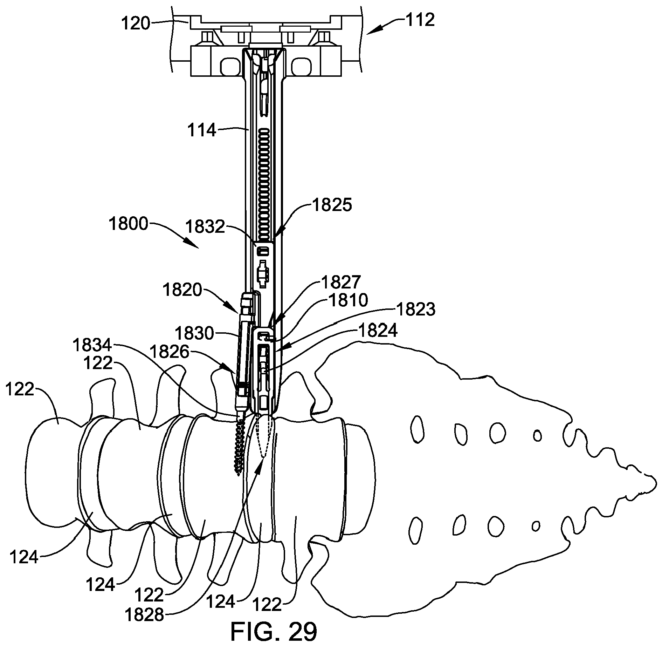

An example retractor stabilization system 1800 which may include or be utilized in conjunction with the retractor assembly 112 is depicted in FIG. 29. The retractor stabilization system 1800 may be configured to be used in a spinal procedure when the subject (e.g., a patient or other suitable subject) is in a prone position to facilitate stabilizing the retractor assembly 112 relative to the subject or in other suitable procedures when the subject is in the prone position or other positions. One example procedure in which the retractor stabilization system 1800 may be utilized is an extreme lateral interbody fusion (XLIF) prone procedure, but the retractor stabilization system 1800 may be utilized in other suitable procedures. Additionally or alternatively, the retractor stabilization system 1800 may be utilized in surgeries in which the subject is in a position other than a prone position (e.g., a lateral decubitus position, etc.) including, but not limited to, XLIF procedures in which the subject is in a non-prone position.

The retractor stabilization system 1800 may include a retractor assembly 112 configured to establish an operative corridor to a spine of a subject and one or more shim assemblies (e.g., one or more stabilizers). The one or more shim assemblies may include one or more shim bodies, one or more attachment components, or both. In one example operation, the one or more shim bodies may be placed in a posterior blade (e.g., the posterior blade 114 or other suitable posterior blade) of a retractor assembly (e.g., the retractor assembly 112 or other suitable retractor assembly) and the attachment component may be inserted through a shim body and into vertebral tissue at a cranial or caudal side of the posterior blade. Additionally or alternatively, the one or more shim assemblies may be engaged with one or more retractor blades other than the posterior blade. The attachment component may be configured to provide fixation between the retractor assembly and a vertebral body of the subject and prevent or mitigate undesired retractor assembly movement from gravitational forces, instrument impaction, etc. throughout a surgical procedure.

The stabilization system 1800, as depicted in FIG. 29, may include a first shim assembly or shim (e.g., a first stabilizer) 1810. The first shim 1810 may be configured to releasably couple to the retractor assembly (e.g., by sliding along a blade of the retractor assembly 112 or couple to the retractor assembly in one or more other suitable manners) and engage intervertebral disc tissue of the subject while the retractor assembly is establishing an operative corridor. The first shim 1810 may have similar features to or different features than the features of the shims discussed herein that are configured to engage intervertebral disc tissue.

The first shim 1810 may include a generally rigid body portion 1823 (e.g., a shim body), a proximal end region 1827 and a distal end region 1828. The distal end region 1828 may include a generally tapered shape designed to be inserted into the disc space 124 (e.g., intervertebral disc tissue of the spine or other suitable disc space). Further, the first shim 1810 may include a tab element 1824 capable of engaging with one or more corresponding grooves (or other similar feature) along an inner-facing surface of a retractor assembly blade (e.g., one of the retractor blades 114, 116, 118). In some examples, the tab element 1824 may also be equipped with an enlarged projection which engages within a corresponding groove provided along an inner surface of a retractor blade 114, 116, 118 of the retractor assembly 112.

The stabilization system 1800 may include a second shim assembly or second shim 1820 (e.g., a second stabilizer). The second shim 1820 may be configured to releasably couple to the retractor assembly (e.g., by sliding along a blade of the retractor assembly 112 or couple to the retractor assembly in one or more other suitable manners) and engage vertebral tissue of the subject while the retractor assembly 112 is establishing an operative corridor. Engagement of the second shim 1820 with the vertebral bodies 122 adjacent the target tissue site may stabilize or at least facilitate stabilizing the retractor assembly 112 relative to the vertebral bodies. The second shim 1820 may include a shim body 1832 configured to couple with the retractor assembly 112 and an attachment component 1834 configured to engage vertebral tissue of the subject.

The shim body 1832 of the second shim 1820 may include an attachment region 1825 designed to engage with one or more corresponding grooves positioned along an inner-facing surface of a retractor blade 114, 116, 118 (e.g., the posterior retractor blade 114 or other suitable retractor blade) of the retractor assembly 112. In some cases, a distal end of the shim body 1832 may be configured to engage a proximal end of the first shim 1810 coupled to the retractor blade 114, 116, 118 at a location distal of a location at which the shim body 1832 is releasably coupled to the retractor blade 114, 116, 118. Additionally, the second shim 1820 may include an offset portion 1826 which is coupled to or may extend from the attachment region 1825.

In some cases, the offset portion 1826 may include a guide 1830 for receiving the attachment component 1834. In one example, the guide 1830, when positioned at the offset portion 1826, may be offset from a central longitudinal axis of the shim body 1832, the retractor blade in which the shim body 1832 is positioned, or both. In some cases, the guide 1830 may have a proximal end and a distal end, where the proximal end is located proximal of a distal end of the shim body 1832 and the distal end of the guide 1830 is distal of the of the distal end of the shim body 1832, as shown in FIG. 29

The attachment component 1834 of the second shim 1820 may be configured to releasably couple to the guide 1830 and extend through a lumen of the guide 1830. The guide 1830 may be configured to direct a distal end of the attachment component 1834 to vertebral tissue 122 of a subject when a retractor blade of a retractor assembly receiving the second shim 1820 is forming an operative/surgical corridor in the subject. When positioned in the guide 1830, the attachment component 1834 may be advanced through the guide 1830 and into vertebral tissue to secure the stabilization system 1800 and the retractor assembly 112 relative to vertebral tissue of the subject. Such positioning of the attachment component 1834 in the vertebral tissue and the second shim 1820 in the retractor assembly 112 may facilitate stabilizing the retractor assembly 112 relative to movement of the spine of the subject, gravitational forces, tool impaction forces, or a combination thereof while the retractor assembly 112 is forming a surgical corridor (e.g., a lateral surgical corridor with the subject in a prone position or other suitable surgical corridor).

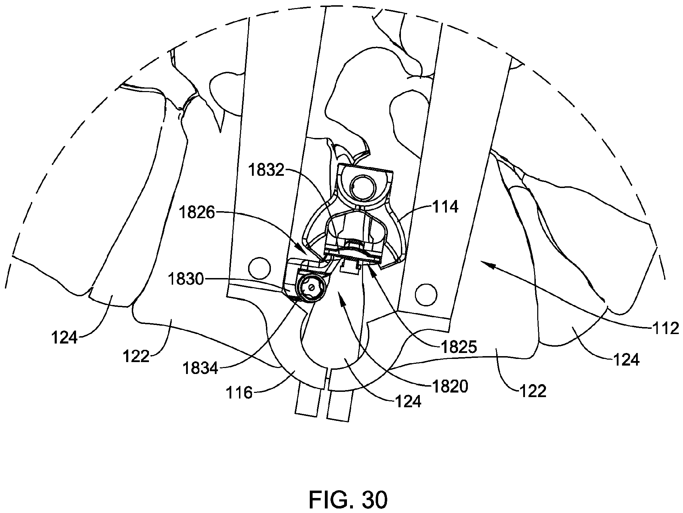

In operation, the second shim 1820 may be inserted into (e.g., slid along grooves of) the posterior blade 114 of the retractor assembly 112 at a surgical site and engaging an intervertebral disc with the first shim 1810. Then, the attachment component 1834 may be advanced into vertebral tissue of the subject during a pre-dilation step, as depicted in FIG. 30 for example, such that the retractor assembly 112 may be secured relative to the vertebrae of the subject before expanding the retractor blades 114, 116, 118 to dilate the operative corridor to a sized desired for performing a surgical procedure.

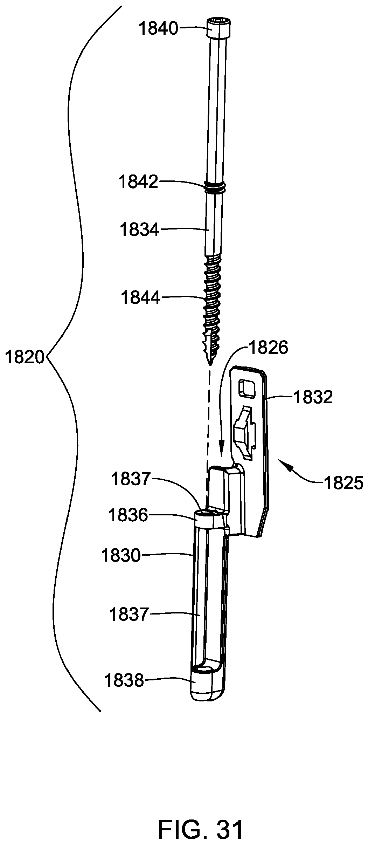

The second shim 1820, in an exploded configuration, is depicted in FIG. 31. As discussed above, the second shim 1820 may include the shim body 1832 and the attachment component 1834.

The shim body 1832 of the second shim 1820 may include the attachment region 1825 and the offset portion 1826. In some cases, the offset portion 1826 may be offset laterally, longitudinally, or axially from the attachment region 1825, as depicted in FIG. 31.

The offset portion 1826 of the second shim 1820 may be or may include the guide 1830 having or defining a lumen extending thru the offset portion 1826 of the second shim 1820. The lumen of the guide 1830 may be configured to extend entirely or at least partially through a length of the guide 1830.

In some cases, the guide 1830 may have one or more portions (e.g., the guide 1830 may be continuous or separated into two or more portions). In one example, the guide 1830 may have a first portion 1836 (e.g., a proximal portion) and a second portion 1838 (e.g., a distal portion) spaced from the first portion 1836, and an intermediate portion 1837 extending between the first and second portions 1836, 1838, as depicted in FIG. 31.

The guide 1830 may be or may include one or more guide tubes having a proximal end and a distal end. When the guide 1830 includes the first portion 1836 and the second portion 1838, first portion 1836 and the second portion 1838 may each be a tube defining the lumen configured to receive an attachment component 1834, where the tube of the first portion 1836 may be connected to the tube of the second portion 1838 by the intermediate portion 1837 (e.g., an open or non-tubular intermediate portion. In some cases, first and second portions 1836, 1838 or other tubular portions may be partial tubes that do not have a full circular cross-section or lumen.

The lumen(s) of the guide 1830 be configured in any suitable manner to receive the attachment component 1834. Although not required, the lumen of the guide 1830 may have one or more features configured to engage or support the attachment component 1834 in one or more positions relative to the guide 1830 as the attachment component 1834 is inserted into or through the guide 1830. In one example, the lumen of the first portion 1836 of the guide 1830 (e.g., proximate a proximal end of a tube of the guide 1830) may include threads 1837 configured to engage threads or a surface of a first portion 1842 of the attachment component 1834. The coupling between the first portion 1836 and the attachment component may be configured to secure the attachment component 1834 relative to the guide 1830 (e.g., in a loading position or other suitable position) such that a vertebra engaging portion of the attachment component 1834 is entirely or at least partially covered by the guide 1830 (e.g., the second portion 1838) as the second shim is inserted into the subject with the retraction assembly 112.

The lumen at the second portion 1838 of the guide 1830 may be configured in any suitable manner. In some cases, the second portion 1838 may have a length and may be positioned relative to the first portion 1836 of the guide 1830 such that the second portion 1838 entirely or at least partially covers the bone engaging portion of the attachment component 1834 while the first portion 1836 of the guide 1830 engages the attachment component 1834. In one example, the second portion 1838 may be positioned proximate a distal end of the guide 1830 and may have a length configured to cover at least a distal tip of the attachment component 1834 when the first portion 1836 of the guide 1830 is engaging the attachment component 1834 to facilitate inserting the second shim 1820 into the body of a subject without the distal tip of the attachment component 1834 engaging body tissue of the subject.

The attachment component 1834 may be any suitable type of attachment component configured to pass through the guide 1830 and into vertebral tissue of the subject (e.g., pass through cortical/compact bone, cancellous bone, or both of the vertebral tissue). Example suitable attachment components 1834 include, but are not limited to, k-wires, screws, and/or other attachment components configured to engage vertebral tissue and couple to the shim body 1832. In one example, the attachment component 1834 may be considered a screw and may include threads.

In some cases, the attachment component 1834 may be any suitable size and shape configured to extend through the guide 1830. In one example, the attachment component 1834 may be elongated and have a length long enough to extend through the guide 1830, extend into vertebral tissue a predetermined distance deep enough to resist pullout of the attachment component 1834 from the vertebral tissue, and create a space between a proximal end of the first portion 1836 of the guide 1830 and a distal end of a proximal head 1840 of the attachment component 1834 when the attachment component 1834 is inserted in the vertebral tissue the predetermined distance.

In some cases, the proximal head 1840 of the attachment component 1834 may have a first outer diameter and an elongated portion of the attachment component extending distally from the proximal head 1840 may have a second outer diameter that is less than the first outer diameter in all or at least some locations along the elongated portion. The first outer diameter of the attachment component 1834 may be greater than an inner diameter of the lumen at a proximal of the guide 1830 and the second outer diameter of the attachment component 1834 may be less than the inner diameter of the lumen at the proximal end of the guide 1830. Such a configuration may facilitate preventing over insertion of the attachment component 1834 into the vertebral tissue of the subject. In some case, the proximal end of the guide 1830 may be configured to be adjustably spaced from a distal end of the proximal head 1840 (e.g., a transition between the first outer diameter and the second outer diameter of the attachment component) when the attachment component is fully engaging the vertebral tissue (e.g., inserted in the predetermined distance and is in an inserted position), as discussed in greater detail below.

In some cases, the attachment component 1834 may have the first portion 1842 (e.g., a guide coupling portion or proximal portion) and a second portion 1844 (e.g., a vertebral coupling portion or distal portion). The first portion 1842 of the attachment component 1834 may be configured to engage the first portion 1836 of the guide 1830. In one example, the first portion 1842 of the attachment component may include threads having a first pitch or configuration configured to engage threads 1837 of the first portion 1836 of the guide 1830 to couple the attachment component 1834 to the guide 1830 and shim body 1832 during insertion of the second shim 1820 or the retraction assembly 112.

The second portion 1844 of the attachment component 1834 may include threads having a second pitch or configuration configured to releasably engage vertebral tissue of the subject. The first pitch of the threads of the first portion 1842 and the second pitch of the threads of the second portion 1844 may be the same pitch or may be a different pitch, as desired.

The threads of the second portion 1844 may be any suitable length for engaging vertebral tissue and securing the attachment component 1834 in the vertebral tissue while preventing or mitigating pullout or unintentional movement of the retractor assembly (e.g., beyond a suitable amount of play, as discussed herein). In some cases, the threads of the second portion 1844 may extend proximally from a distal tip of the attachment component 1834 any suitable distance. Example suitable distances include, but are not limited to, up to a width of a vertebra in which the attachment component 1834 is to be inserted, a length of 0.5" to 2.25", and/or other suitable distances. In one example, a length or distance of the threads extending proximally from the distal tip of the attachment component 1834 may be or may be about 1.0", but this is not required and the length or distance may be shorter or longer than 1.0".

The threads of the attachment component 1834 may be continuous or spaced from one another. In one example, the threads of the second portion 1844 may be spaced distally (e.g., at or proximate a distal end of the attachment component 1834) from the threads of the first portion 1842, as depicted in FIG. 31.

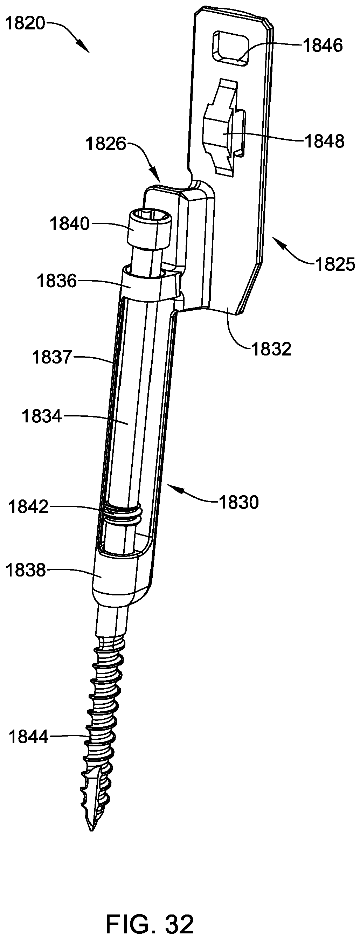

Perspective, front, back, right side, left side, top, and bottom views, of the second shim 1820 depicted in FIG. 31 are depicted in FIGS. 32-38, respectively. The attachment component 1834 depicted in FIGS. 32-38 is shown in a vertebral engaging or inserted position (e.g., the inserted position as if the second portion 1844 of the attachment component 1834 were engaging vertebral tissue), with a proximal portion of the attachment component 1834 extending proximal of the proximal end of the guide 1830.

The perspective view of the second shim 1820 depicted in FIG. 32 shows the offset portion 1826 of the second shim 1820 as being offset in a forward and lateral direction from the attachment region 1825 where the shim body 1832 is configured to engage a retractor blade. The offset position of the offset portion 1826 of the second shim 1820 in the forward and lateral directions may facilitate positioning the second shim 1820 in the retractor blade without the guide 1830 interfering with the retractor blade in which the second shim 1820 is inserted or other retractor blades of the retractor assembly, while also aligning the guide 1830 with a vertebra adjacent an intervertebral disc with which the first shim may engage (e.g., see FIG. 30.

The shim body 1832 is depicted in FIG. 32 as including engagement components. For example, the shim body 1832 may include an engagement feature 1846 (e.g., an aperture, as depicted in FIGS. 32-38, a dimple, a bump, etc.) configured to engage with a shim placement tool to facilitate removing and/or adjusting the second shim 1820 once the second shim 1820 is engaged with a retractor blade. Additionally or alternatively, the shim body 1832 may include a tab 1848 (e.g., a flexible engagement tab and/or other suitable tab) configured to engage a shim placement tool. In some cases, the tab 1848 may have a trailing edge or back side configured to engage the retractor blade in which the second shim 1820 is inserted to prevent unintended backout of the second shim 1820 relative to the retractor blade, but this is not required. Other suitable engagement features for the second shim 1820 are contemplated.

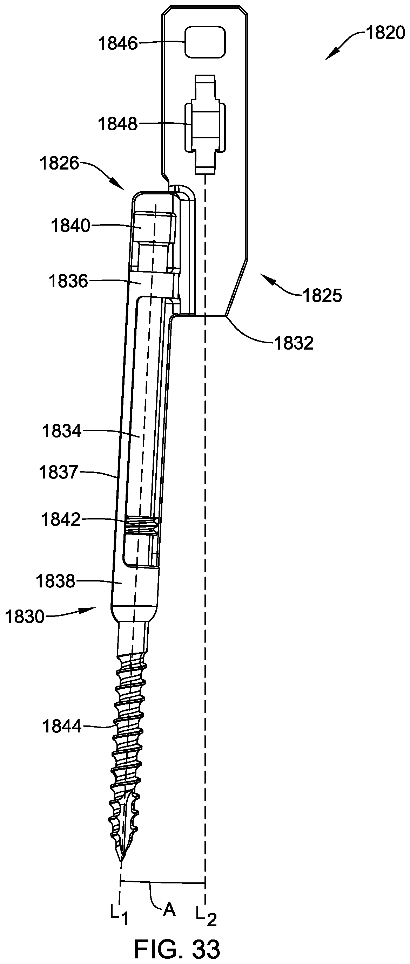

The front view of the second shim 1820 depicted in FIG. 33 shows the shim guide 1830 having a first longitudinal axis L₁ (e.g., a central longitudinal axis of the guide 1830 or other suitable axis) and the shim body 1832 having a second longitudinal axis L₂ (e.g., a central longitudinal axis of the attachment region 1825 of the shim body 1832 or other suitable axis), where the first longitudinal axis L₁ is at an angle A relative to the second longitudinal axis L₂. The angle A between the first longitudinal axis L₁ and the second longitudinal axis L₂ may be set to any desired angle suitable for facilitating insertion of the second shim 1820 in a retractor blade or surgical site and for aligning the guide 1830 and the attachment component 1834 with vertebral tissue when the second shim 1820 is positioned in the retractor blade. The angle A may be set based on any one or more of a variety of factors including, but not limited to, a length of the attachment component 1834, an intended location of the guide 1830 relative to a vertebral body or tissue when the second shim 1820 is in intervertebral tissue, etc. Angle A may be a value, for example, from 0 degrees to 10 degrees, 1 degree to 5 degrees, or other suitable value configured to align the guide 1830 with vertebral tissue adjacent intervertebral tissue engaged by the first shim 1810 or a retractor blade in which the second shim 1820 is positioned. In one example, the angle A may be 0 degrees, such that the first longitudinal axis L₁ is parallel to the second longitudinal axis L₂. In another example, the angle A may be 3 degrees, as depicted in FIG. 33. Other suitable values for angle A are contemplated.

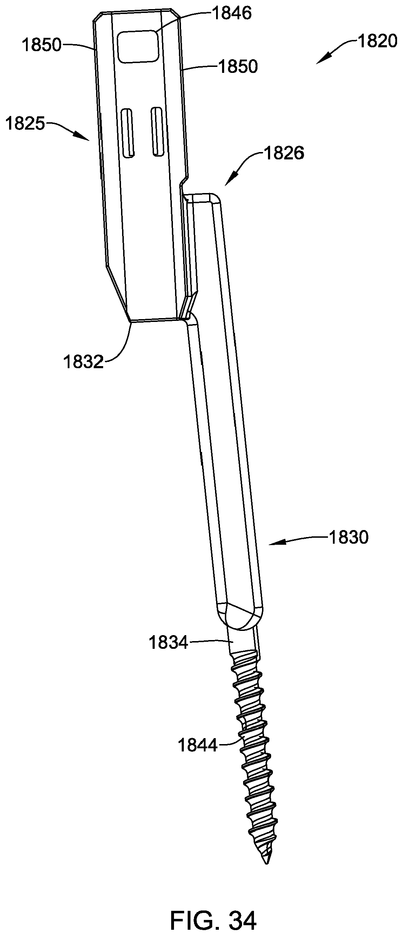

The back view of the second shim 1820 depicted in FIG. 34 shows the shim body 1832 with elongated engagement members 1850. The elongated engagement members 1850 may be configured to slideably engage elongated slot members that run along an inside surface of a retractor blade to facilitate positioning the second shim 1820 in a retractor blade. The elongated engagement members 1850 may take on any suitable shape or configuration configured to slideably engage the slot members of the retractor blade. The elongated engagement members 1850 may extend along a length of the shim body 1832 at the lateral sides of the attachment region 1825 of the shim body 1832, as depicted in FIG. 34, but other configurations are contemplated.

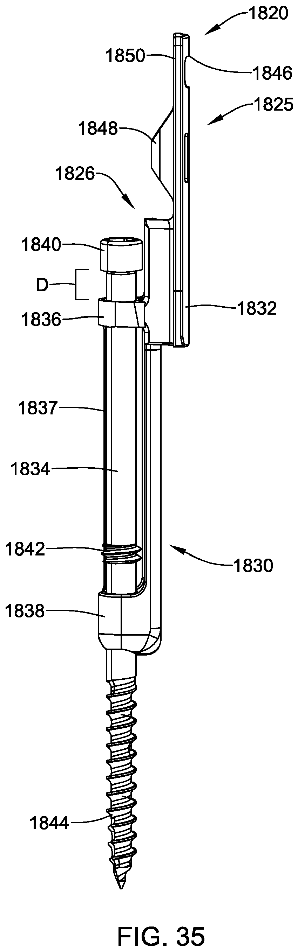

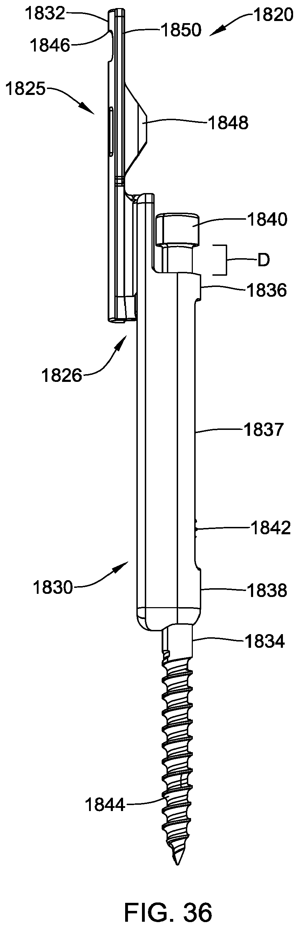

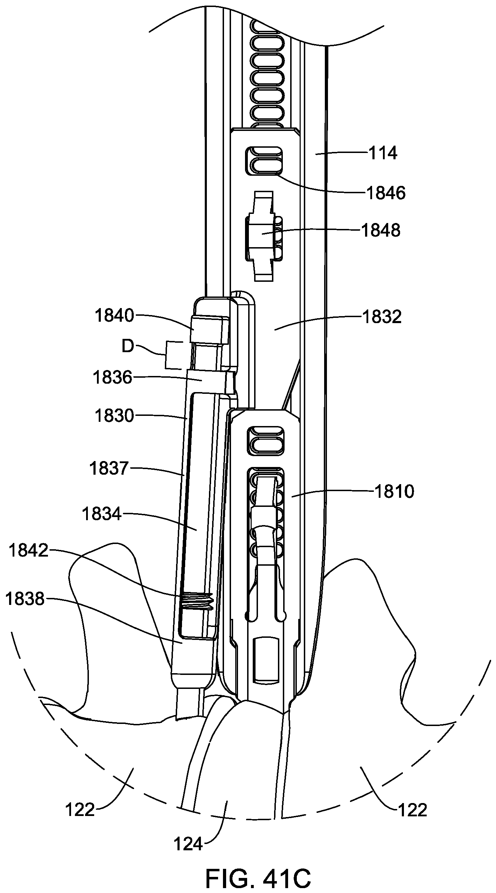

The right and left side views of the second shim 1820 are depicted in FIGS. 35 and 36. As mentioned above, the attachment component 1834 is in a vertebral engaging position within the guide 1830 of the second shim 1820. The guide 1830 and the attachment component 1834 may be configured such that a distal end of the proximal head 1840 of the attachment component 1834 is spaced a distance D from a proximal end of the first portion 1836 of the guide 1830 when the attachment component 1834 is fully inserted in the guide 1830 (see FIG. 41C showing the attachment component 1834 fully inserted in the guide 1830 and in vertebral tissue). The distance D between the distal end of the proximal head 1840 and the proximal end of the first portion 1836 may facilitate mitigating pullout of the attachment component 1834 from the vertebral tissue due to allowing for some movement of the guide 1830, shim body 1832, and thus the retractor assembly relative to the attachment component 1834 (e.g., relative to or along an axis of the attachment component 1834) engaging vertebral tissue.

Distance D between the distal end of the proximal head 1840 and the proximal end of the first portion 1836 of the guide 1830 may be any suitable value to allow for some movement of the retractor assembly relative to the engagement component due to gravitational forces, spinal movement, tool impaction during surgery, etc., while still providing sufficient retractor assembly stabilization. For example, the value of Distance D may be 1 millimeter (mm) to 5 mm or other suitable distance. In one example, the distance D may be 3 mm, but other suitable values for distance D are contemplated.

The guide 1830 may be in-line with the shim body 1832, offset in an anterior direction relative to the shim body 1832, or offset in a posterior direction relative to the shim body 1832, as desired. Additionally or alternatively, a longitudinal axis of the guide 1830 (e.g., the longitudinal axis L₁ or other suitable longitudinal axis) may be posteriorly or anteriorly offset at any suitable angle relative to an axis at or parallel to a longitudinal axis of the shim body 1832 (e.g., the longitudinal axis L₂ or other suitable longitudinal axis) including, but not limited to, an angle between 0 to 10 degrees in an anterior or posterior direction relative to an axis at or parallel to the longitudinal axis of the shim body 1832. As depicted in FIGS. 35 and 36, the guide 1830 is parallel to the shim body 1832 (e.g., offset at 0 degrees relative an axis at or parallel to the longitudinal axis of the shim body 1832) and offset in an anterior direction relative to the shim body 1832, but this is not required. The posterior or anterior offset of the guide 1830 relative to the shim body 1832 may be set based on any one or more of a variety of factors including, but not limited to, a length of the attachment component 1834, an intended location of the guide 1830 relative to a vertebral body when the second shim 1820 is in a vertebral body, etc.

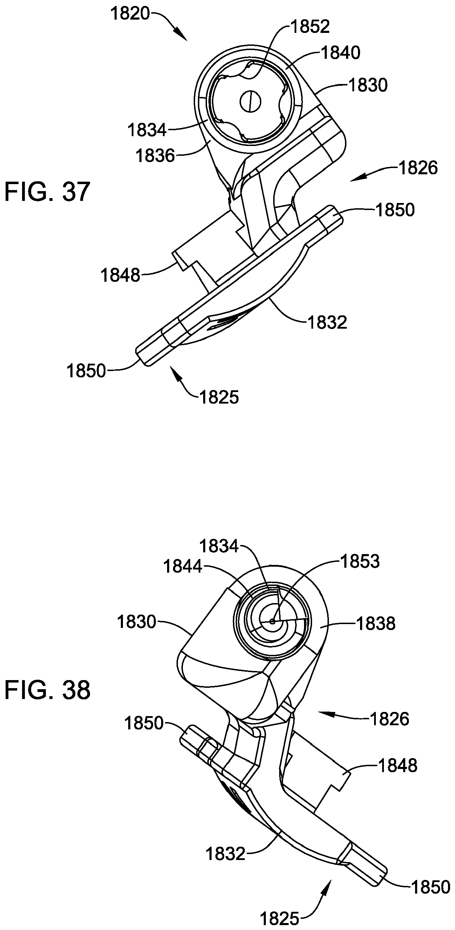

The top and bottom views of the second shim 1820 are depicted in FIGS. 37 and 38. The guide 1830 of the offset portion 1826 is positioned forward of and to a side of the shim body 1832, as depicted in FIGS. 37 and 38.

The proximal head 1840 of the attachment component 1834 may include a socket 1852, as depicted in FIG. 37, configured to receive a tool for inserting the attachment component 1834 through the guide 1830 and into vertebral tissue or for removing the attachment component 1834 from the vertebral tissue and the guide 1830. Other suitable head configurations are contemplated including, but not limited to, heads having a hexagon shape, heads having a star shape, heads configured to receive a Phillips-head screw driver, heads configured to receive a flat-head screw driver, or other suitable head configurations.

In some cases, utilizing a socket 1852 in the proximal head 1840 may facilitate insertion or removal of the attachment component 1834. In one example, the socket 1852 may facilitate insertion or removal of the attachment component 1834 by allowing for use of a tool that may grip the proximal head by applying a radially outward pressure on the proximal head 1840 when the tool is inserted therein, where the radially outward pressure grips the inner surface of the proximal head 1840 and allows the attachment component 1834 to be pushed or pulled as the tool is turned. Additionally or alternatively, a lip of a tool inserted into the proximal head 1840 of the attachment component 1832 may engage an overhang of the proximal head 1840 such that a mechanical interference occurs between the proximal head 1840 and the tool when the tool is rotated in a removal direction (e.g., a counter-clockwise direction) to allow a user to apply a pulling force to the attachment component 1834 as the tool is rotated.

A distal end of the attachment component 1834 may include a distal tip 1853, as depicted in the bottom view of FIG. 38, to which the threads of the second portion 1844 of the attachment component 1834 may taper. In some cases, the distal tip 1853 and threads may be configured to engage and bore into vertebral tissue upon rotational movement of the attachment component 1834, axial pressure on the attachment component 1834, or both.

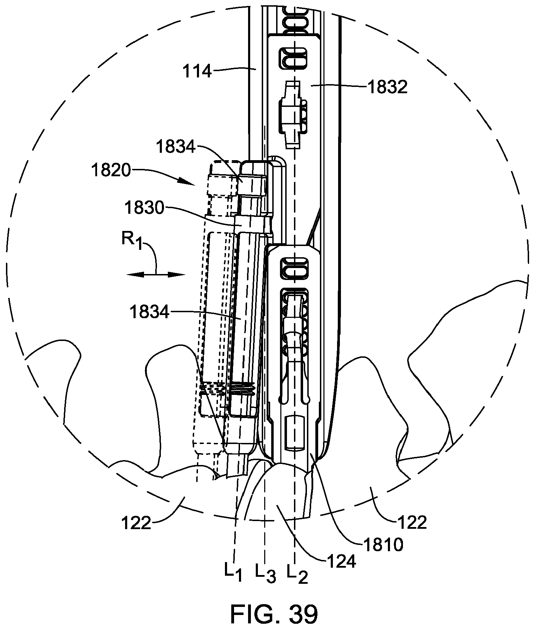

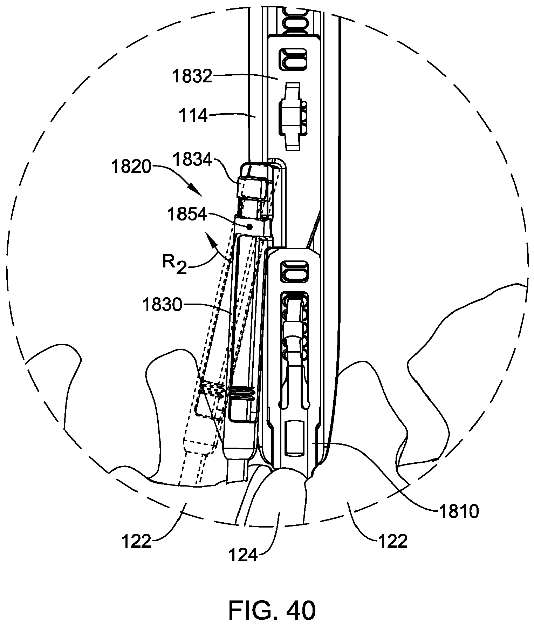

The guide 1830 of the second shim 1820 may be fixed relative to the shim body 1832, as depicted in FIGS. 32-38, the retractor blade in which the guide may be positioned, or both, but this is not required. In some cases, the guide 1830 of the second shim 1820 may be configured to adjust or move (e.g., translate, rotate, pivot, etc. or any combination of movements) relative to the shim body 1832, as depicted, for example, in FIGS. 39 and 40, or the retractor blade in which the shim body 1832 is configured to be positioned. In some cases, movement of the guide 1830 relative to the second shim 1820 may facilitate allowing a user (e.g., a surgeon or other suitable user) to place the attachment component 1834 in vertebra of the subject at a desired location of the vertebra.

The guide 1830 of the second shim 1820 depicted in FIG. 39 may be configured to translate or rotate laterally inward or outward relative to at least a portion of the shim body 1832 (e.g., the translation or rotation of the guide 1830 are represented by the broken lines and arrow Ri in FIG. 39). Although other configurations are contemplated, the guide 1830 may be configured to translate or rotate about a third longitudinal axis L₃ that may be parallel to one or both of first longitudinal axis L₁ of the guide 1830 and the second longitudinal axis L₂ of the shim body 1832.

To facilitate the laterally inward or outward translating or rotating movement of the guide 1830 relative to the shim body 1832, the guide 1830 may be coupled to the shim body 1832 in any suitable configuration that facilitates the movement. For example, the guide 1830 may be coupled to the shim body 1832 via a hinge, a flexible member, a ball joint with or without a restriction on degrees of freedom, or other suitable adjustable connection. In one example, the guide 1830 may be coupled to the shim body 1832 with a hinge member having two or more pre-set positions, but other adjustable coupling techniques are contemplated.

In some cases, the guide 1830 of the second shim 1820 may be configured to pivot or rotate relative to the shim body 1832, as depicted in FIG. 40 (e.g., the pivoting of the guide 1830 are represented by the broken lines and arrow R₂ in FIG. 40). Although other configurations are contemplated, the guide 1830 may be configured to pivot about one or more axes. In one example, the guide 1830 may be configured to pivot about one or more axes that are perpendicular to the longitudinal axis L₂ of the shim body 1832, but this is not required.

To facilitate the pivoting of the guide 1830 relative to the shim body 1832, the guide 1830 may be coupled to the shim body 1832 or other portion of the second shim 1820 in any suitable configuration that facilitates the movement. For example, the guide 1830 may be coupled to the shim body 1832 or other portion of the second shim via a pin, a ball joint with or without a restriction on degrees of freedom, or other suitable adjustable connection. In one example, the guide 1830 may be coupled to the shim body 1832 or other portion of the second shim 1820 with a pivot pin 1854 (although the pivot pin 1854 is depicted in FIG. 40, the pivot pin 1854 would be behind the attachment component 1834), but other adjustable coupling techniques are contemplated.

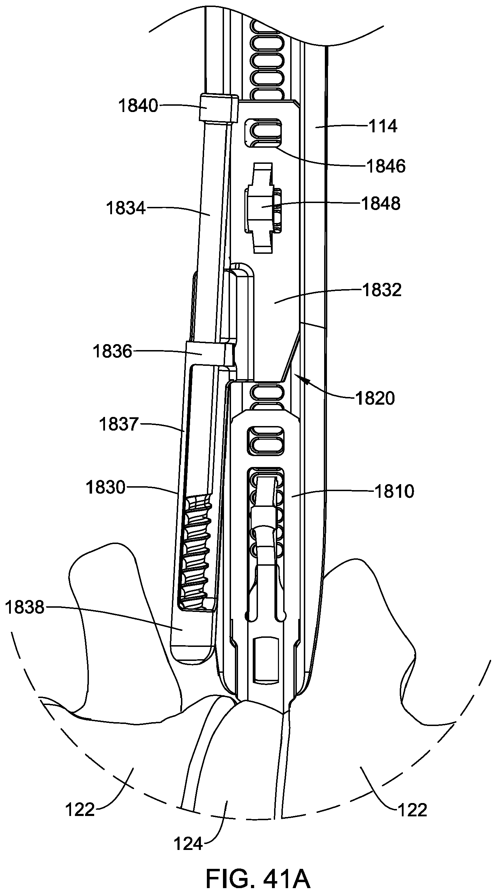

FIGS. 41A-C depict a technique for inserting the second shim 1820 (e.g., a second stabilizer for the retractor assembly) into a surgical site along a posterior retractor blade 114. The second shim 1820, as depicted, for example, in FIG. 41A may include the guide 1830, the shim body 1832, and the attachment component 1834 inserted into the guide 1830 to an insertion/withdrawal position (e.g., a loading/unloading position) in which the distal end of the attachment component 1834 is covered by the second portion 1838 of the guide 1830. As discussed above, in the insertion/withdrawal position, the threads at the first portion 1842 (not shown in FIG. 41A) of the attachment component 1834 may engage the first portion 1836 of the guide 1830 such that a distal end of the threads at the vertebral coupling portion 1844 of the attachment component 1834 are covered by the guide 1830 (e.g., the second portion 1838 of the guide 1830). Although the second shim 1820 is depicted as being inserted into the posterior retractor blade 114, the second shim 1820 may be inserted into one or more other retractor blades of a retractor assembly used for creating an operative corridor.

Initially, the first shim 1810 (e.g., a first stabilizer for the retractor assembly) may be engaged with an intervertebral disc tissue 124 of a subject (e.g., where the subject may be in a prone position) and the first shim 1810 may be coupled with the posterior retractor blade 114 of a retractor assembly. Once the first shim 1810 is positioned within the intervertebral disc tissue 124, a surgical corridor (e.g., a lateral surgical corridor or other suitable surgical corridor) within the subject may be formed using the retractor assembly. With the attachment component 1834 in the insertion/withdrawal position and the posterior retractor blade 114 and the first shim 1810 positioned at or in the intervertebral tissue 124 and forming the surgical corridor within the subject, the shim body 1832 may engage grooves of the posterior retractor blade 114 and may be advanced (e.g., slid or advanced in one or more other suitable manners) distally along the posterior retractor blade 144 until a distal end of the shim body 1832 is proximate or contacts a proximal end of the first shim 1810. In some cases, a shim placement tool may engage the engagement feature 1846 or the tab 1848 of the second shim 1820 and may be advance to direct the second shim 1820 along the posterior retractor blade 114. Other suitable techniques for positioning the second shim 1820 in the posterior retractor blade 114 are contemplated.

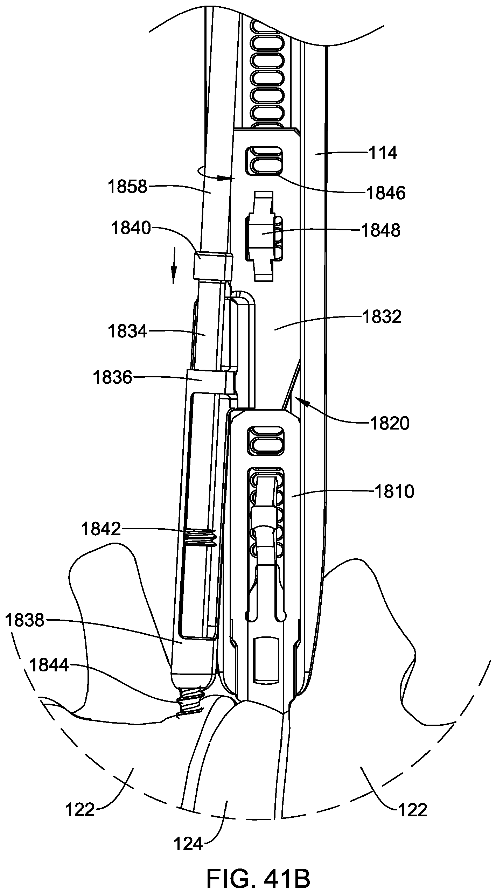

Once the second shim 1820 has been positioned within the posterior retractor blade 114 with a distal end of the shim body 1832 proximate a proximal end of the first shim 1810, the attachment component 1834 may be advanced into vertebral tissue 122, as depicted in FIG. 41B to engage the vertebral tissue 122. In some cases, an attachment component positioning tool 1858 may be utilized to advance the attachment component 1834 into the vertebral tissue and secure the retractor assembly with respect to the spine of the subject. With the attachment component positioning tool 1858 engaging the proximal head 1840 of the attachment component 1834, an axial force (e.g., axial force along the guide 1830) and a rotational force may be applied to the attachment component 1834 to thread the attachment component through the threads of the guide 1830 and advance the attachment component 1834 into the vertebral tissue 122.

Once the attachment component 1834 has been fully inserted into vertebral tissue 122 (e.g., is in the inserted position), a spacing having a distance D, as depicted, for example, in FIG. 41C, may remain between the proximal end of the first portion 1836 of the guide 1830 and the distal end of the proximal head 1840 of the attachment component 1834 (e.g., a distal shoulder of the proximal head 1840 or transition from the first diameter to the second diameter of the attachment component 1834) to facilitate play or slight movement of the retractor assembly due to tool impaction or other forces during a surgical procedure. In some cases, a value of the distance D may be 3 mm, but other suitable values are contemplated. With the attachment component 1834 fully inserted into vertebral tissue 122, the retractor blades of the retractor assembly may be expanded radially outward create an surgical or operative corridor of a desired size.

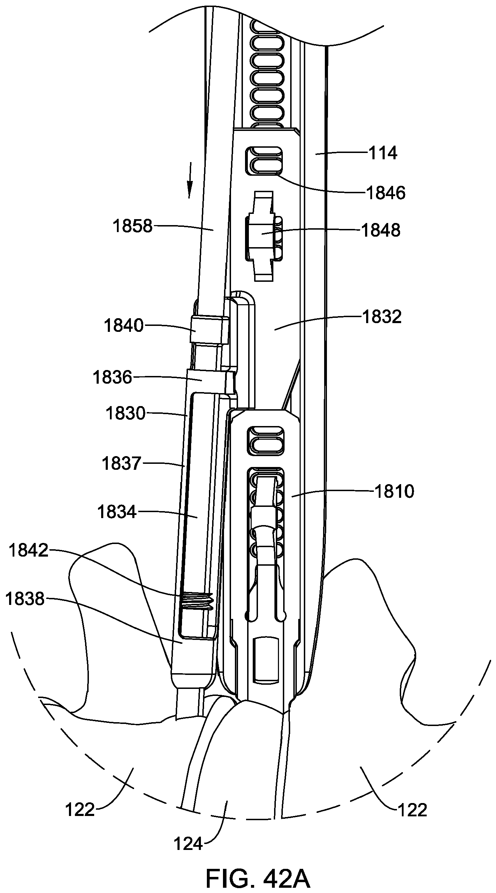

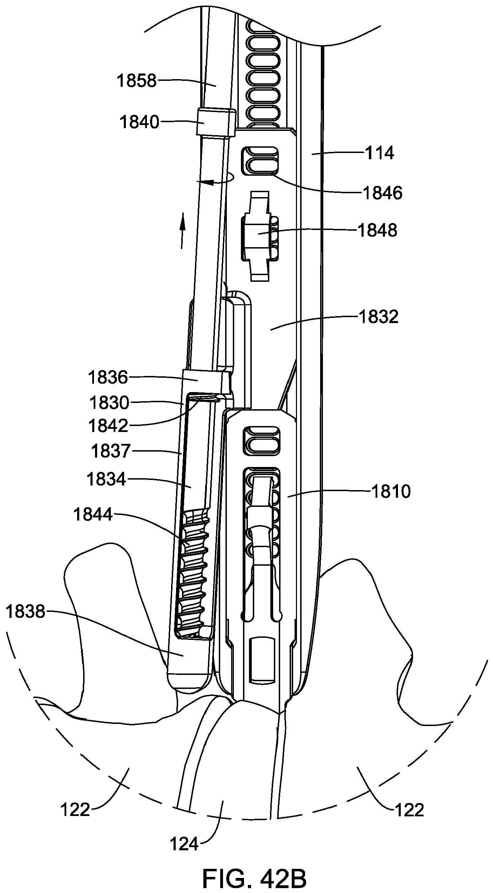

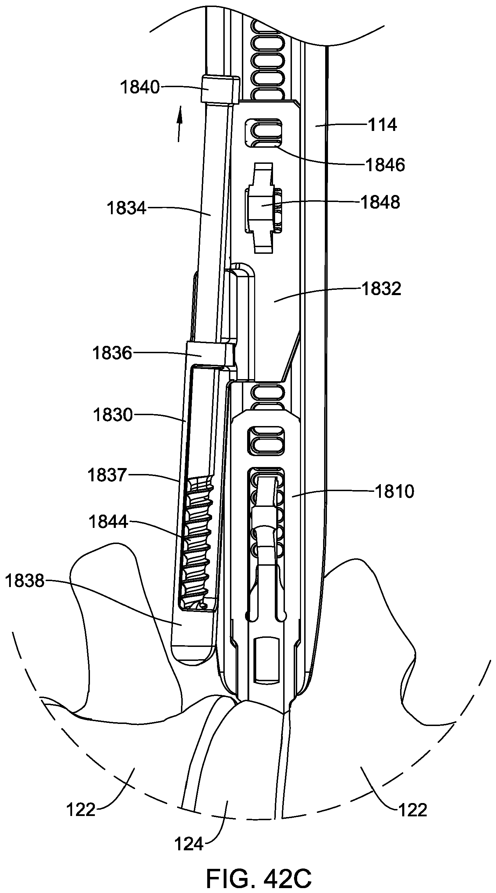

After a surgical procedure utilizing the desired surgical or operative corridor has been completed or at one or more other suitable times, the second shim 1820 may be removed from the vertebral tissue or the retractor assembly. FIGS. 42A-C depict a technique for removing the second shim 1820 (e.g., the second stabilizer) from a surgical site along a posterior retractor blade 114. With the attachment component 1834 engaging the vertebra tissue of the subject in the inserted position, the attachment component positioning tool 1858 may engage the proximal head 1840 of the attachment component 1834 and may be rotated (e.g., in a direction that is the reverse of a direction the attachment component positioning tool 1858 was rotated for insertion of the attachment component 1834) to cause the threads of the second portion 1844 of the attachment component 1834 to backout or disengage the vertebral tissue 122. In some cases, the attachment component positioning tool 1858 may be configured to have a distal end that can put radial outward pressure on the proximal head 1840 (e.g., via the socket 182) such that pulling on the attachment component positioning tool 1858 (e.g., in an axial direction or other suitable direction) may assist in withdrawing the attachment component 134 from the vertebral tissue 122 until the threads of the first portion 1842 engage the first portion of the guide 1830 and the distal tip (e.g., the distal tip 1853, not shown in FIGS. 42A-C) is covered by the second portion 1838 of the guide 1830. When the distal tip of the attachment component 1834 is covered by the second portion 1838 of the guide 1830, the attachment component 1834 may be in an insertion/withdrawal position, as depicted, for example, in FIG. 42B.

Optionally, after the attachment component is withdrawn from vertebra tissue 122, the retractor assembly may be adjusted to reduce a size of the operative corridor defined by the retractor blades of the retractor assembly. With the attachment component 1834 in the insertion/withdrawal position and, optionally, the operative corridor in a reduced size, the second shim 1820 may be withdrawn or removed from the posterior retractor blade 114 or the surgical corridor (e.g., a lateral surgical corridor). In some cases, the shim placement tool may positioned in the operative corridor and engaged with the engagement feature 1846, the tab 1848 or both. Once engaged with the second shim 1820, the shim placement tool may be withdrawn from the operative corridor and the second shim 1820 may slide proximally along the retractor blade, out of the operative corridor, and out of engagement with the retractor assembly.

Although other configurations are contemplated, example configurations of shim bodies for the shims discussed herein and example couplings between the shims and a retractor blade of a retractor assembly are disclosed in U.S. Patent Application No. 11/137,169, filed on May 25, 2005, and titled SURGICAL ACCESS SYSTEM AND RELATED METHODS, which is hereby incorporated by references in its entirety for any and all purposes.

Although other configurations are contemplated, example configurations of shim bodies for the shims discussed herein, example couplings between the shims and a retractor blade of a retractor assembly, and tools for adjusting and/or removing shims relative to retractor blades are disclosed in U.S. Patent Application No. 13/821,224, filed on January 27, 2014, and titled SURGICAL ACCESS SYSTEM AND RELATED METHODS, which is hereby incorporated by references in its entireties for any and all purposes.

Although other configurations are contemplated, example configurations of tools for adjusting and/or removing shims relative to retractor blades are disclosed in U.S. Patent Application No. 29/508,821, filed on November 11, 2014, and titled COMBINED INTRADISCAL INSERTION TOOL AND INTRADISCAL SHIM, which is hereby incorporated by references in its entireties for any and all purposes.

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The detailed description should be read with reference to the drawings in which similar elements in different drawings are numbered the same. The drawings, which are not necessarily to scale, depict illustrative embodiments and are not intended to limit the scope of the disclosure.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. An assembly, comprising:
a shim body; and
a guide coupled to the shim body,
wherein the shim body is a surgical shim body configured to releasably couple to a retractor blade of a retractor assembly, and
wherein the guide is configured to receive an attachment component and direct a distal end of the attachment component to vertebral tissue of a subject when the shim body is coupled to the retractor blade and the retractor blade is forming a surgical corridor in the subject.

2. The assembly of claim 1,
wherein the guide is offset from a central longitudinal axis of the shim body.

3. The assembly of claim 1,
wherein the guide has a proximal end and a distal end and a guide tube extends at least part of a length from the proximal end to the distal end.

4. The assembly of claim 1,
wherein the guide has a proximal end, a distal end, and threads proximate the proximal end.

5. The assembly of claim 1,
wherein the guide has a proximal end and a distal end, and
wherein the proximal end of the guide is proximal of a distal end of the shim body and the distal end of the guide is distal of the distal end of the shim body.

6. The assembly of claim 1, further comprising:
the attachment component,
wherein the attachment component is configured to releasably couple to the guide.

7. The assembly of claim 6,
wherein the attachment component is configured to releasably engage the vertebral tissue of the subject to stabilize the retractor assembly relative to movement of a spine of the subject while the retractor assembly is forming a lateral surgical corridor with the subject in a prone position.

8. The assembly of claim 6,
wherein the attachment component is threaded.

9. The assembly of claim 8,
wherein the attachment component includes threads at a distal end of the attachment component, the threads at the distal end of the attachment component are configured to releasably engage the vertebral tissue of the subject.

10. The assembly of claim 8,
wherein the attachment component includes threads at a location proximal of a distal end of the attachment component, the threads at the location proximal of the distal end of the attachment component are configured to engage threads on the guide.

11. The assembly of claim 8,
wherein the attachment component includes a first set of threads at a distal end of the attachment component and a second set of threads at a location proximal of the first set of threads.

12. The assembly of claim 11,
wherein the first set of threads have a first threaded configuration and the second set of threads have a second threaded configuration.

13. The assembly of claim 11,
wherein the first set of threads are configured to releasably engage threads of the guide, and
wherein when the first set of threads are releasably engaged with the threads of the guide, a distal end of the second set of threads are covered by the guide.

14. The assembly of claim 6,
wherein the guide includes a proximal end defining an inner diameter of a guide tube of the guide tube, and
wherein a proximal end of the attachment component defines a first outer diameter greater than the inner diameter of the guide tube.

15. The assembly of claim 14,
wherein the attachment component includes a second outer diameter that is less than the inner diameter of the guide tube and a proximal end of the guide is configured to be adjustably spaced from a transition between the first outer diameter and the second outer diameter of the attachment component when the attachment component is engaging the vertebral tissue of the subject.
